(19) Europäisches Patentamt
European Patent Office
Office européen des brevets

(11) EP 1 505 394 A1

(12) EUROPEAN PATENT APPLICATION

(43) Date of publication:
09.02.2005 Bulletin 2005/06

(51) Int Cl.7: G01N 33/84, G01N 33/68

(21) Application number: 03077492.1

(22) Date of filing: 08.08.2003

(84) Designated Contracting States:
AT BE BG CH CY CZ DE DK EE ES FI FR GB GR HU IE IT LI LU MC NL PT RO SE SI SK TR
Designated Extension States:
AL LT LV MK

(71) Applicant: Kreatech Biotechnology B.V.
1032 LG Amsterdam (NL)

(72) Inventors:
- Talman, Eduard Gerhard
2353 RV Leiderdorp (NL)
- Veuskens, Jacky Theo Maria
3500 Hasselt (BE)
- O'Flaherty, Martina Catherine
2135 DZ Hoofdorp (NL)
- van der Hoeven, Paulus Cornelis Joseph
3911 WJ Rhenen (NL)

(74) Representative:
Prins, Adrianus Willem, Mr. Ir. et al
Vereenigde,
Nieuwe Parklaan 97
2587 BN Den Haag (NL)

# (54) Platinum complexes for differential mass labelling

(57) The invention relates to the use of a platinum complex for creating a defined shift in the molecular weight of said entity in order to facilitate mass spectrometric analysis of said entity, to methods for rendering chemical or biological entities distinguishable by mass spectrometry as well as to methods for mass spectrometric analysis of the chemical or biological entities. Further, the present invention relates to a set of at least two platinum complexes of different molecular weight, to platinum complexes comprising different stable isotopes, to chemical or biological entities obtained by a method of the invention and to a kit of parts supporting the use and/or methods of the invention

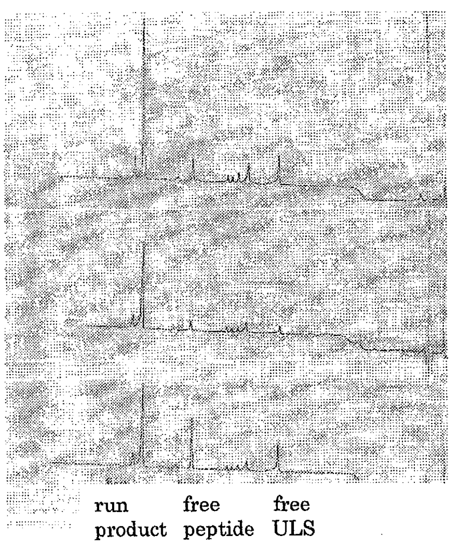


Figure 13: HPLC of LWMR peptide labeled with monofunctional ULS-d0-BOC (37) and monofunctional ULS-d4-BOC (27) and as a mixture. Absorbance was at 220nm on a C18 (4.6mm) 5u column.

EP 1 505 394 A1

## Description

### FIELD OF THE INVENTION

The invention relates to the use of platinum complexes for creating a defined shift in the molecular weight of said entity in order to facilitate mass spectrometric analysis of said entity, to methods for rendering chemical or biological entities distinguishable by mass spectrometry as well as to methods for mass spectrometric analysis of chemical or biological entities. Further, the present invention relates to a set of at least two platinum complexes of different molecular weight, to chemical or biological entities obtained by a method of the invention and to a kit of parts supporting the use and/or methods of the invention.

### BACKGROUND OF THE INVENTION

It is generally recognized that the level of expression or the state of activity of proteins in a biological cell cannot be derived solely from the expression level of mRNA gene transcripts. This is because the rate of synthesis and the half-life as well as the activity of proteins is for a large part controlled by post-transcriptional processes.

In order to understand the functioning of biological systems, and to evaluate the effect of disease states or drug administration, it is essential that the composition of the proteome as well as qualitative and quantitative changes therein can be determined. The human proteome comprises more than 100 000 proteins with widely diverse characteristics, naturally occurring at concentration levels covering up to nine orders of magnitude. These figures illustrate some of the challenges faced by proteome researchers. The large-scale (ultimately global) analysis of proteins expressed in a cell or tissue has been termed proteome analysis (Pennington et al., 1997, Trends Cell Bio 7:168-173) or proteomics. Compared to genome analysis, proteome analysis is facing specific challenges.

A broad range of technologies is available to identify proteins and map their cellular interactions. In order to unravel the nature of an unknown protein and to assess its function, a number of subsequent steps is generally involved (Patterson and Aebersold, 2003, Nat. Genet. 33 Suppl:311-23).

A first step is formed by proteome display and mapping technologies. The most widely used procedure to obtain protein maps or fingerprints is two-dimensional gel electrophoresis (2DE) (Rabilloud, 2000, Anal Chem. 72(1): 48A-55A). In 2DE, proteins are separated by their isoelectric point (PI) in one dimension followed by molecular weight (MW) separation in the second dimension. The separated proteins can be visualized using general stains such as Coomassie blue, silver stains, fluorescent dyes, or radio labeling (Patton, 2002, J Chromatogr B Analyt Technol Biomed Life Sci. 771(1-2):3-31). The generated complex patterns of spots require advanced image analysis for further processing. Usually two or multiple protein profiles, representing e.g. different cell stages, are compared. Differentially expressed protein spots of interest are excised from gel for post-separation analysis. In this way the changes in the proteome due to an alteration in the cell's state can be visualized and the characteristic changes in protein expression can be pinpointed.

A subsequent second step is represented by protein identification technologies, predominantly Mass Spectrometry (MS). A mass spectrometer is essentially a combination of an ionization module, a mass analyzer and a detector and measures the mass-to-charge (m/z) ratio of ionized analytes (e.g. peptides or proteins). The analytes can be charged either by fast atom bombardment (FAR), electron spray ionization (ESI), matrix-assisted laser desorption ionization (MALDI) or atmospheric pressure chemical ionization (APcI). The mass analyzer is the most flexible part of the mass spectrometer. Since an electric field will deflect charged particles, and the energy potential can be converted to inertial movement based on the mass and the potential, the mass analyzer steers certain masses to the detector based on their mass-over-charge ratios (m/z) by varying the electrical field potentials. The analyzer can be used to stabilize a narrow range of m/z or to scan through a range of m/z to catalog the ions present. Several types exist, include time-of-flight, ion trap, and quadrupole mass analyzers. The detector simply records the charge induced when an ion passes by or hits a surface.

Because a mass spectrometer is a non-specific detector, specific interfaces are often used to link a mass spectrometer to a gas (GC) or liquid chromatograph (LC) for feeding separate analytes to the MS. A tandem mass spectrometer (MS-MS or $MS^n$) is one that is capable of multiple rounds of mass spectrometry. For example, one mass analyzer can isolate one peptide from many entering a mass spectrometer. A second mass analyzer then stabilizes the peptide ions while gas collides with them, causing them to fragment. A third mass analyzer then catalogs the fragments produced from the peptides. This process, called collision induced dissociation, is the basis of many experiments in proteomics. To date, various mass spectrometers are available, each featuring its specific advantages for certain applications (Gygi and Aebersold, 2000, Curr Opin Chem Biol. 4(5):489-94; Mann et al., 2001, Ann. Rev. Biochem. 70:437-473).

A final step in assessing the functional analysis of a proteins involves technologies such as surface plasmon resonance (SPR), which can provide detailed information on biomolecular interactions, e.g. of known proteins and their

targets. The phenomenon of SPR occurs in a thin metal film at an optical interface under conditions of total reflection of polarized light at a specific angle. The procedure measures subtle changes in optical resonance, and is sensitive to the refractive index of the medium on the opposite side of this film. Changes in refractive index occur when molecules bind to, or dissociate from a biomolecule that has been attached to the metal surface. In this way, real time binding activity profiles are generated with association or dissociation characteristics of the interaction (Green et al., 2000, Biomaterials 21(18):1823-35; Rich and Myszka, 2000, Curr Opin Biotechnol. 11(1):54-61; McDonnell, 2001, Curr Opin Chem Biol. 5(5):572-7).

Technological advancements have generally been aimed at improving the sensitivity and quantification of detection of the various proteins. Moreover they have been aimed at providing methods that can make 2DE obsolete, i.e. to become gel-independent. The complexity of 2DE patterns and the inherent biases against low abundant, small (less than 10 kDa) or poorly soluble (e.g. membrane bound) proteins have triggered the development of techniques that allow (semi) quantitative measurements of proteins in a proteome directly (Regnier et al., 2002, J Mass Spectrom. 37(2):133-45; Bauer and Kuster, 2003, Eur J Biochem. 270(4):570-8).

A very suitable approach that circumvents the use of 2DE is the ICAT (isotope-coded affinity tags) procedure developed by Gygi et al. in 1999 (Nat. Biotechnol. 17(10):994-9). This procedure is capable of rapidly comparing two different global protein expression profiles by MS based on differential labeling with stable isotopes. The first generation ICAT reagent consisted of i) a thiol-specific protein reactive group (iodoacetamide) capable of labeling cysteine (Cys) residues of proteins, ii) two different linker moieties containing either eight hydrogen ($^1$H) atoms (d0, light isotope) or eight deuterium ($^2$H) atoms (d8, heavy isotope) for differential labeling of two populations of proteins, and iii) an affinity tag, mostly biotin, that enables selective isolation of labeled peptides.

The ICAT procedure itself involves the reduction and alkylation of the cysteine side chains in a complex mixture of proteins, whereby the proteins of one cell state are labeled with the d0-labeled tag and the proteins of a second cell state (e.g. a disease state) are labeled with the d8 form of the tag. The two mixtures are then combined and subjected to proteolytic digestion, in order to provide fragments amenable to MS analysis. The resultant complex mixture of proteolytic peptides is purified by affinity column chromatography to pull out the labeled sub-set of peptides. The sample is then analyzed by a combination of LC-MS, for providing the quantitative information or the abundance of proteins based on the relative abundance of the d0 and d8 isotopes, and LC-MS-MS, for providing the qualitative information based on the molecular weight of the peptide and the amino acid sequence information.

Improper co-elution of peptides labeled with the heavy ICAT reagent versus peptides labeled with the light ICAT, and poor recovery of affinity tag captured labeled peptides led to the development of the second generation ICAT reagent. These ICAT reagents are composed of i) a thiol-specific protein reactive group (iodoacetamide) capable of labeling cysteine (Cys) residues of proteins, ii) a linker moiety containing a defined number of either carbon atoms (light) or carbon isotopes (heavy) for differential labeling of two populations of proteins, iii) an affinity tag, mostly biotin, that enables selective isolation of labeled peptides, and iv) acid cleavable site which allows removal of the biotin portion of the ICAT reagent tag prior to MS and MS-MS analysis. The protein reactive group covalently links the isotope-coded affinity tag to the protein by alkylation of free cysteines.Because the ICAT technique is based on labeling of the cysteine residue(s) of a protein, proteins that do not contain a cysteine will not be detected with ICAT. Moreover, the cysteine residue must be located in a tractable peptide; that is, a peptide that results from the proteolytic digestion with a molecular weight (MW) of approximately between 600 and 3500 Da. Peptides outside of this MW range will either not contain a sufficient number of amino acids to provide the necessary qualitative information for protein identification, or will be so large as to not be amenable to MS-MS fragmentation by collisionally activated dissociation. Moreover, the cysteine-based ICAT tags would not yield information on changes in the proteome based on post-translational modification such as phosphorylation, unless the modification fortuitously occurred in the cys-containing peptide.

Various alternative methods have been developed to differentially label proteins by both chemical and metabolic labeling procedures (Goshe and Smith, 2003, Curr Opin Biotechnol. 14(1):101-9). The chemical methods are based on classical protein modifications of individual peptides or protein trypsin digests. For instance, lysine (Lys) residues may be labeled at the C-terminus by using a heavy derivative of 2-methyloxy-1H-imidazole containing four $^2$H atoms (d4) (Peters et al., 2001, Rapid Commun Mass Spectrom. 15(24):2387-92). Lys residues may also be labeled by differential guanidination using O-methyl-isourea in a process termed mass-coded abundance tagging (MCAT; Cagney and Emili. 2002. Nat Biotechnol. 20(2):163-70). Methionine (Met) residues may for instance be labeled by using activated esters of (d0/d4)-nicotinic acid (Shen et al., 2003. Molec Cell Proteomics 23:..-...). Cys residues may be labeled by differential alkylation using N-(d0/d5)-ethyl-iodoacetamide. Primary amines of peptides can be differentially labeled using N-acetoxy-(d0/d3)-succinimide derivatives (Chakraborty and Regnier, 2002).

In general, however, the above labeling methods require 2DE separation or - in the case of ICAT - affinity chromatography purification, to reduce the complexity of the mixtures before MS analysis.

Also, the labeling chemistry involved lacks robustness, partly due to the instability of the chemical reagent and its undesired cross-reacting properties, which leads to heterogeneity in the signal pairs obtained. These factors hamper the utilization of differential labeling methods in proteome research, especially in case of (automated) high-throughput applications.

Currently, there is a need for robust procedures for differential labeling of peptides that can support both large-scale analysis in proteome research as well as routine (diagnostic) testing. It is therefore desirable that such labeling procedures offer improved selectivity of the labeling reaction, i.e. that offer the possibility of labeling specific reactive sites of an entity, preferably while essentially preventing the loss of native characteristics, *e.g.* 3D structure, activity, avidity, *etc.* while at the same time facilitating mass spectrometric analysis of the labeled entities. It is further desirable that upon differential labeling, the products can be separated by alternative means than by affinity chromatography before being subjected to mass spectrometry. A further desired improvement over the current technology is the omission of the purification step prior to MS. Instead, it would be advantageous if differentiation between labeled and non labeled molecules can be made during MS-MS. It is an objective of the present invention to provide a method that fulfils one or more of these needs. A problem to be solved resides in the difficulty of providing labeling methods that result in a bond between entity and label that can survive the ionisation process of mass spectrometry.

## SUMMARY OF THE INVENTION

The present inventors have now found that platinum complexes comprising a platinum complex having at least one reactive moiety for attachment to a bio-organic molecule can advantageously be used as tags in methods for differential labeling of biomolecules and that such platinum complexes provide improvements over existing reagents for differential expression labeling and circumvent the various problems of the prior art reagents.

The present invention now provides for the use of a platinum complex, having at least one reactive moiety for attachment to a chemical or biological entity, for creating a defined shift in the molecular weight of said entity in order to facilitate mass spectrometric analysis of said entity or of a sample comprising said entity.

Also, the present invention provides a method for rendering a chemical or biological entity distinguishable by mass spectrometry, said method comprising the step of differentially labeling said entity with at least one platinum complex.

In another aspect, the present invention provides a method for mass spectrometric analysis of a chemical or biological entity, said method comprising the steps of differentially labeling said entity with at least one platinum complex and analyzing said entity by mass spectrometry.

In preferred embodiments of methods of the present invention said chemical or biological entities originate from different samples.

In yet other preferred embodiments of methods of the present invention said step of differentially labeling said entities is performed with at least two platinum complexes of different molecular weight, said difference in molecular weight being ≥1 Da.

In preferred embodiments of methods of the present invention the platinum complexes display same labeling characteristics, same properties in liquid chromatography (LC), e.g. same hydrophobicity or hydrophilicity, same platinum oxidation state, preferable +2.

In yet other preferred embodiments of methods of the present invention the platinum isotope fingerprint may be used as an identification tool of labeled entities.

The molecular weight difference between said platinum complexes may be due to the presence or absence of specific isotopes, atoms, groups of atoms, molecules, or groups of molecules in any part of the platinum complexes, or ligands, spacers, reactive moieties and/or markers attached thereto.

In preferred embodiments of the invention said molecular weight difference between said platinum complexes is due to the presence of different stable isotopes in said complexes, e.g. wherein a first platinum complex comprises a light isotope and at least a second platinum complex comprises a heavy isotope.

The use and methods of the invention are supported by both *trans* and *cis*-platinum complexes. In preferred embodiments of the invention, the platinum complexes are *cis*-platinum complexes comprising an inert bidentate moiety as a stabilizing bridge.

The stable isotopes that cause the difference in molecular weight may be comprised in any part of the platinum complexes. Preferably, they are comprised in said bidentate moiety.

In another aspect, the present invention provides a set of said at least two platinum complexes of different molecular weight.

A further aspect of the present invention relates to a kit of parts comprising a set of at least two platinum complexes of different molecular weight. Preferably, the difference in molecular weight results from the presence of at least one different stable isotope between said platinum complexes.

## DETAILED DESCRIPTION OF THE INVENTION

A "chemical or biological entity" as used herein is to be interpreted as something that comprises one or more

sulphur containing reactive sites and/or one or more nitrogen containing reactive sites.

"Entity" further relates to a micro-organism, a virus or a pribn, or to a material comprising one or more of said sulphur reactive or nitrogen reactive types of reactive sites, or a product made thereof, such as a micro-array, a microtitre plate, a test strip or a test tube. In particular an entity relates to an inorganic or organic compound, including a bio-organic compound.

A "bio-organic compound" as used herein refers to a biological carbon containing compound. Also, a bio-organic compound refers to a compound capable of inducing or affecting an action in a biological system, *e.g.* by inducing or affecting a therapeutic or prophylactic effect, an immune response, a metabolic process *etc.*

With a "platinum complex", a platinum moiety is meant that can be used to couple a marker to an entity. A preferred platinum complex as used in this invention is a platinum complex to which ligands are bound.

The term "labeling" is used herein to refer to the process of coupling a platinum complex to an entity, said complex optionally including a marker or affinity label.

The term "differential labeling" is used herein to refer to the labeling reaction resulting in an unequal distribution of the marker between reactive sites, between entities or between samples. Differential labeling may result in one entity having different markers at distinct reactive sites, e.g. an entity having its S- and N-reactive sites differentially labeled. Differential labeling may also result in identical entities from one sample having different markers or having different marker densities, e.g. one sample may have identical proteins that are differentially labeled. Also, differential labeling may result in identical entities from two samples having different markers or having different marker densities. Such a type of differential labeling is very suitable for comparative analysis of proteomes between cells.

A "marker", "tag", or "label" as used synonymously herein may be any moiety that can be attached to the platinum complex or the entity, and that can be used to detect, monitor or visualize the entity.

An "affinity label" as used herein may be any moiety functioning as a molecular handle that selectively binds covalently or non-covalently, to a capture reagent, and that can be attached to the platinum complex. An affinity label represents a specific type of marker. Binding of the affinity label to a capture reagent facilitates isolation of entities, e. g. peptides, substrates or reaction products tagged or labeled with the affinity label.

A "residue" of a compound as used herein should be interpreted as the compound itself or as part of a larger entity, *e.g.* an amino acid residue in a protein.

A "different stable isotope" is herein defined as an alternative isotope of an atom that is stable and is not the isotope of said atom which is most abundant in nature. In the case of C (carbon) $^{13}C$ (1.07%) would be a different stable isotope, as apposed to the normally occurring $^{12}C$ (98.93%). In case of Pt (platinum) $^{192}Pt$ (0.79%), $^{194}Pt$ (32.9%), $^{196}Pt$ (25.3%), and $^{198}Pt$ (7.2%) are different stable isotopes as opposed to the normally occurring and $^{195}Pt$ (33.8%).

An entity linked to a platinum complex may be referred to as a Pt-S adduct (when attached to a sulphur containing reactive site), to a Pt-N adduct (when attached to a nitrogen containing reactive site), or in general to a Pt-adduct.

A sulphur containing reactive site may hereafter be referred to as a S-reactive site, and a nitrogen containing reactive site may hereafter be referred to as N-reactive site.

It is well known in the art that a chemical or biological entity may be labeled with a detectable marker to detect, visualize, quantify or monitor the entity *e.g.* in chemical, biological or medical research or diagnosis. A wide variety of labeling methods are known from the art (for a review see Hermanson, 1996, Bioconjugate techniques, Academic Press, ISBN 0-12-342335-X). Many factors may play a role in choosing a particular detectable marker and a particular method of labeling. Such factors include the nature of the entity, the conditions of the labeling reaction, the sensitivity during the labeling reaction, the specificity towards the entity and the detection limits of the labeled entity.

Methods using platinum complexes to label bio-organic molecules have been contemplated for a very long time. Platinum complexes may react with a variety of reactive moieties on an entity and various types of detectable marker moieties can be adhered to ionic platinum.

The use of a *cis*-platinum complex has for instance been described in WO96/35696 and WO02/069898, which discloses a method for linking bio-organic molecules and markers through *cis*-platinum complexes. In these complexes, two co-ordination sites are occupied by either ends of a stabilizing bridge, such as an ethylene diamine group. Platinum complexes described in any of these publications are incorporated herein by reference. *Cis*-platinum complexes are suitable for linking labels to several kinds of bio -organic molecules, such as peptides, polypeptides, proteins, and nucleic acids. Methods using *trans*-platinum complexes have also been reported (EP 0 870 770) as suitable for labeling a variety of bio-organic molecules and linking of labels thereto.

The reactivity of platinum complexes towards a variety of reactive sites is a benefit in many applications, since it may allow fast labeling reactions and an excellent sensitivity towards a wide variety of entities.

The present inventors have found that a particular advantage of the use of platinum complexes as linkers in labeling reactions over such linkers as N-hydroxysuccinimide (NHS) and maleimide is that platinum complexes are routinely applicable in mass spectrometry applications. Furthermore, the specific composition and ratio between the

most abundant Pt atom and its different stable isotopes can provide for a defined tool in identifying labeled entities in mass spectrometry.A further advantage of the use of platinum complexes in labeling reactions is that, depending on the reactive moieties used, such complexes may support the labeling of a wide variety of chemical entities.

Another advantage is that proteins may be labeled by a suitable platinum complex at histidine (His), methionine (Met) and/or cysteine (Cys) residues. This provides for the possibility to label more side groups in a peptide chain and thus to achieve higher labeling densities. This may also allow for the labeling of additional amino acid residues in a peptide chain.

Not all biological proteins and/or peptides comprise cysteine, and cysteine labeling will allow for the detection of only 85% of the proteins in the proteome. The possibility to label methionine residues will allow for the detection of 97% of the proteins in the proteome. In combination, the use of the labeling complexes of the present invention now provides for a proteome coverage of 98.35%. Thus, the possibility of choosing new target amino acids represents an important advancement in proteome research.

Moreover, the specificity of the labeling reaction with platinum complexes may be controlled such as to discriminate between labeling of sulphur containing reactive sites and nitrogen containing reactive sites in a chemical or biological entity. Therefore, by using the platinum complex labeling reagents according to the present invention one can direct the labeling of an entity towards a specified reactive site within an entity or a group of entities that together comprise a variety of reactive sites In one embodiment of a use of a platinum complex according to the invention, the chemical or biological entities in a sample may be differentially labeled according to a method described in European patent application 1 262 778.

In a preferred embodiment the use of a platinum complex according to the invention comprises the differential labeling of chemical or biological entities present in two different samples of which the composition is to be compared. A preferred use includes the measurement of the protein expression profile, or proteome in a test sample, in order to compare the results thereof with corresponding results from a reference sample.

Yet another advantage of the use of platinum complexes in differential labeling procedures of proteins is that there is no interference with the subsequent trypsin digestion. This is especially advantageous in labeling procedures of cellular proteomes that are subjected to trypsin digestion for the provision of peptides of suitable length for MS analysis.

Examples of preferred platinum complexes suitable for the present invention are *cis*- or *trans*-platinum complexes of the formula [Pt(II)(X1)(X2)(A)(D)] or a cis-platinum complex of the formula [Pt(II)(X3)(A)(D)].

Herein, Pt represents platinum (Pt), A and D represent the same or different reactive moieties, respectively involved in the complexation of the platinum complex to a marker and the linking of the platinum complex to the entity. The entities, X1 and X2 represent the same or different inert moieties, and X3 represents an inert moiety that may act as a stabilizing bridge, *e.g.* a bidentate ligand.

A structural representation of some examples of such platinum complexes is shown below:

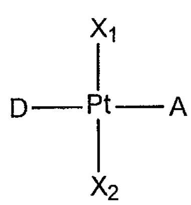

formula 1a

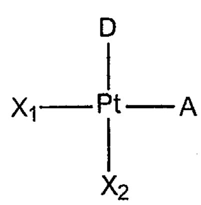

formula 1b

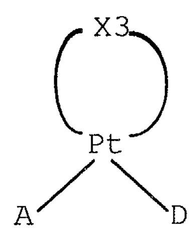

formula 1c

Yet, other preferred platinum complexes suitable for the present invention are platinum complexes having more then one reactive moiety capable of linking to an entity. In a subset of platinum complexes the inert moiety, label, and/or affinity tag may be combined.

A platinum(II) complex, for use in a method of the invention can be prepared via any method known in the art. References can for example be found in Reedijk *et al*. (Structure and Bonding, 67, pp. 53-89, 1987). The preparation of some *trans*- platinum complexes is disclosed in EP 0 870 770. Further preparation methods can be found in WO 96/35696 and WO 98/15564. Methods described in any of these publications are incorporated herein by reference. In a preferred embodiment of the invention platinum complexes are prepared according to the spacer - *tert* butoxycarbonyl / NHS - label pathway as described by Heeterbrij *et al* (ChemBioChem 2003, 4, 573-583).

The reactive moieties (A, D) of a platinum complex are preferably good leaving ligands. A platinum complex, wherein A and/or D are independently chosen from the group of Cl-, $NO_3$-, $HCO_3$-, $CO_3^{2-}$, $SO_3^{2-}$, $ZSO_3$-, $I^-$, Br-, F- ,

acetate, carboxylate, phosphate, ethylnitrate, oxalate, citrate, a phosphonate, ZO-, and water has been found to be particularly suitable for use in a method according to the invention. Z is defined herein as a hydrogen moiety or an alkyl or aryl group having from 1 to 10 carbon atoms. Of these ligands, $Cl^-$ and $NO_3$- are most preferred.

Although Pt(IV) complexes can be used in the present invention Pt (II) complexes are preferred because these are better soluble in aqueous solutions and display favorable labeling kinetics. Any type of inert moiety X3 may be chosen. Inert as used herein indicates that the moiety remains attached to the platinum complex during the labeling process and thereafter without chemically reacting with an entity. A platinum complex comprising one or two inert moieties chosen from the group of $NH_3$, $NH_2R$, NHRR', NRR'R" groups, wherein R, R' and R" preferably represent an alkyl group having from 1 to 6 carbon atoms have been found to be particularly suitable for use in a method of the present invention. $H_2NCH_3$ is a particularly preferred inert moiety. An alkyl diamine, wherein the alkylgroup has 2 to 6 carbon atoms is a preferred bidentate inert moiety in a *cis*-platinum complex (*e.g.* X3 in formula 1c). In a particularly preferred embodiment X3 represents ethylene diamine. Other preferred inert moieties are heterocyclic diamines and heteroaryl diamines such as but not limited to heterocyclic-1,2-diamines and heteroaryl-1,2-diamines, and aryl diamines. Particularly preferred platinum complexes for use in the present invention include cis[Pt(en)$Cl_2$], cis[Pt(en)Cl($NO_3$)], cis[Pt(en)$(NO_3)_2$], trans[Pt$(NH_3)_2Cl_2$], trans[Pt$(NH_3)_2$Cl($NO_3$)], and trans[Pt$(NH_3)_2(NO_3)_2$].

In principle, any type of nitrogen containing reactive site or sulphur containing reactive site of an entity may be labeled using a method according to the invention. Preferred reactive sites include reactive sites comprising a primary amine, a secondary amine, a tertiary amine, an aromatic amine, a thiol, a thioether, a sulfide, a disulfide, a thioamide, a thion, an amide, an imide, an imine, an iminoether, or an azide. Examples of entities that can be labeled are entities chosen from the group of amino acids (preferably methionine, cysteine, histidine, lysine, and tryptophan), peptides, oligopeptides, polypeptides, proteins, immunoglobulins, enzymes, synzymes, phospholipides, glycoproteins, nucleic acids, nucleosides, nucleotides, oligonucleotides, polynucleotides, peptide nucleic acids, peptide nucleic acid oligomers, peptide nucleic acid polymers, amines, aminoglycosides, nucleopeptides, and glycopeptides. Preferably in accordance with the invention, the entity is chosen from the group of amino acids, peptides, oligopeptides and polypeptides.

The creation of a defined shift in the molecular weight of a chemical or biological entity upon binding of a platinum complex will ultimately result from the attachment of the platinum complex thereto. Therefore, said shift will essentially coincide with an change in the mass of the entity that is proportional to the mass of the platinum complex minus the weight of the leaving portion of a leaving group. Said mass change can be determined by mass spectrometric analysis.

A method for rendering chemical or biological entities distinguishable by mass spectrometry according to the invention comprises the differential labeling of said entities with at least one platinum complex, wherein suitable platinum complexes are as described herein. The use herein of two different platinum complexes, each having its own labeling specificity, allows for the possibility of differentially labeling different entities with in one sample.

Apart from the possibility to label entities originating from one sample, i.e. said entities occurring as a mixture, the chemical or biological entities may also originate from different samples. The differential labeling may for instance comprise the labeling of entities in a test sample, without labeling the entities in the reference sample or *vice versa*. Identical entities present in both samples then become distinguishable by mass spectrometry as a result of the defined shift in the molecular weight of the entities in the test sample. Alternatively, the differential labeling may also comprise the labeling of entities in both samples. In such instances, rendering said entities distinguishable by mass spectrometry will involve the differentially labeling of said entities with at least two platinum complexes of different molecular weight.

A method for differential labeling may be performed in a wide variety of buffered solution and over a wide pH range. Suitable buffered solutions include all commonly used buffers such as TRIS/Glycine and phosphate buffers. Detergents such as Tween-20, Triton X-100 or SDS may be present in the labeling mixture. The presence of salts, such as sodium, potassium or ammonium salts of chloride, nitrate, sulphate or phosphate hardly affects the labeling reaction.

The reaction parameters for the differential labeling reaction may also be chosen such that an entity is differentially labeled and include the choice for a specific pH value. The pH as used herein should be interpreted as the pH value of water at 20 °C. In general, the formation of Pt-S adducts is pH independent whereas formation of Pt-N adducts is pH dependent. In a preferred embodiment one or more S-reactive sites are selectively labeled over one or more nitrogen containing sites by making use of the pH.

As a guideline, one may choose the pH of the labeling reaction at a pH below the lowest pKa of any of an entity's N-reactive sites that should not be labeled, allowing differential labeling of one or more S-reactive sites. As the skilled professional will understand other factors, besides pKa, may play a role, including the influence of the microenvironment in the vicinity of an entity that is to be labeled. In general, S-reactive sites are differentially labeled over N-reactive sites at acidic pH.

In theory, the formation of a Pt-S adducts is a one step process. A reactive group leaves the platinum complex upon S donating an electron pair to platinum. This process, the direct conversion Pt-X into Pt-S, is believed to be pH

independent. On the other hand, N donors require replacement of a reactive group of the platinum complex by oxygen prior to N substitution. First, Pt-X becomes Pt-O and eventual Pt-N. This is a two step scheme in which the first step can be controlled by changing pH. Factors influencing pH of a solution may therefore interfere with Pt-N adduct formation.

The presence of ions may also be used to control the selectivity of the platinum complex for N-reactive sites. In an embodiment one or more leaving ligands, preferably anionic moieties, are used in the inhibition of labeling a platinum complex to a N-reactive site, in order to enhance differentiated labeling of a S-reactive site. Examples of such leaving ligands include $Cl^-$, $NO_3$-, $HCO_3$-, $CO_3^{2}$-, $ZSO_3$-, $SO_3$-, I-, Br-, F-, acetate, carboxylate, phosphate, ethylnitrate, oxalate, citrate, a phosphonate, ZO-, and water. Z is defined herein as a hydrogen moiety or an alkyl or aryl group having from 1 to 10 carbon atoms. Particularly good results have been achieved by using salts comprising an anionic moiety, of which chloride is particularly preferred. The counter ions are preferably alkali cations, alkali earth cations or cations also used to direct the labelling. In a preferred embodiment the total ionic strength of said anionic moieties used in the inhibition of labeling to a N-reactive site is at least 0.1 mol/1. More preferably the total ionic strength is in the range of 0.1 to 0.5 mol/1.

The presence of metal ions, such as transition metal ions, may also be used for selection of the reactive site to be labeled. In particular such ions have been found suitable to prevent or slow down labeling of an S-reactive site or to make a labeled Pt-S adduct labile, so that effectively one or more N-reactive sites are differentially labeled over said S-reactive site. Within a method according to the invention it is also possible to direct the labeling by making use of geometrical isomers of a platinum complex - *e.g.* a *cis*-platinum complex and a *trans*-platinum complex, - such that the platinum complex is specifically labeled to either a sulphur containing reactive site or to a nitrogen containing reactive site.

This form of differential labeling of entities or samples may hold specific advantages when only a subset of entities is to be analyzed.

In addition to the parameters as mentioned above a method according to the invention may further be fine tuned by parameters such as temperature, preferably varied in the range between 0 °C and 120 °C, more preferably in the range between 20 °C and 70 °C; reaction time, commonly in the range between 1 min and 48 hours, preferably in the range between 10 min and 24 hours, more preferably in the range between 25 min and 15 hours; concentration of the reagents, molar ratio of the reagents, overall net charge of the platinum labeling complex, and the like. These parameters may be adjusted depending upon the particular application in any way known in the art. The overall net charge of the platinum labeling complex, for example, affects the specificity of Pt-N adduct formation in histidine at neutral pH. Neutral Pt-complexes, such as fluorescein- and cyanine Pt complexes, form Pt-N adducts whereas positively charged platinum labeling complexes, *e.g.* rhodamine- and dinitrophenol Pt complexes, do not. Positively charged Pt labeling complexes display differential labeling towards N adducts above the isoelectric point of the peptide, protein, and the like. Apart from allowing the selective labeling of N-reactive sites over S-reactive sites or vice versa, a method according to the present invention also makes it possible to differentiate between distinct N-reactive sites or distinct S-reactive sites, by choosing the correct conditions, such as described in European Patent Application 01202007.9.

The above instructions for differential labeling of a single entity may also be employed to obtain the required or optimal labeling for differential labeling of different compounds, and from compounds of different origin.

A method for mass spectrometric analysis of the chemical or biological entities according to the invention must first render such entities distinguishable by MS and thus involves a first step of differentially labeling the entities with at least one platinum complex as described above. Further, such a method involves the step of analyzing the molecular weight of said entities by mass spectrometry. The skilled person is knowledgeable about the various possibilities of MS detection.

Prior to MS detection, entities from different samples may optionally be mixed and the mixture be subjected to MS analysis. The advantage of mixing differentially labeled entities or samples prior to MS analysis is that the MS conditions are equal for all entities tested and the shift in the molecular weight can be easily discerned.

Also, prior to MS detection, labeled entities may be purified from unlabeled entities by e.g. affinity isolation of the affinity tagged materials, or particularly in methods of the present invention by ion exchange chromatography, or by using fluorescent markers in combination with fluorescence-activated cell sorting (FACS). Furthermore, platinum complexes of the present invention offer the opportunity to separate (differential) labeled entities during MS-MS without prior purification.

In methods involving sample comparison, it is therefore preferred that both samples are labeled with platinum complexes of different molecular weight in order to allow a purification step to be performed on both said samples. In this way, purification bias is equal for both samples. As an affinity label any label capable of binding to a capture reagent may be used. In preferred embodiments of the present invention the affinity label is a biotin or dinitrophenol (DNP) and the preferred capture reagent is a strepavidin or avidin and anti-DNP, respectively. After affinity isolation of affinity tagged materials, some of which may be isotopically labeled, the interaction between the affinity label and the capture reagent is disrupted or broken to allow MS analysis of the isolated materials. The affinity label may be displaced from

the capture reagent by addition of displacing ligand, which may be free affinity label or a derivative of said affinity label, or by changing solvent (e.g., solvent type or pH) or temperature conditions or the linking complex may be cleaved chemically, enzymatically, thermally or photochemically to release the isolated materials for MS analysis.

Prior to MS detection, the labeled entities are separated to allow the detection of preselected species of molecules. Prior separation, for instance by liquid chromatography (LC) or gas chromatography (GC), will enable the analysis by MS of a complex mixture of proteins. The skilled person will be able to determine required or optimal separation procedures for the specific application.

A method for mass spectrometric analysis of the chemical or biological entities may comprise the comparative analysis of a test sample and a reference sample. When after differential labeling, the two samples are admixed and the mixture subjected to chromatographic separation followed by mass spectrometric analysis, the entities labeled with the platinum complex will exhibit a predictable molecular weight shift compared to the non-labeled entities. As such, the said method of the invention allows comparative mass spectrometric analysis of the chemical or biological entities between samples.

In order to distinguish entities of a test sample from entities of a reference sample on the basis of their molecular weight, such a method may also comprise the provision of at least two platinum complexes of different molecular weight and the differential labeling of said entities between said samples. In other words, the test sample entities are labeled with a platinum complex of a first molecular weight, and the reference sample entities are labeled with a platinum complex of a second molecular weight. Depending on the resolution required, a difference in molecular weight between the platinum complexes of 1 Da may suffice. Advantageously, the difference in molecular weight between the platinum complexes in said set is more than 2, more advantageously more than 4, preferably more than 6, more preferably more than 8, and even more preferable more than 10 Da.

The purpose of the two platinum complexes with different molecular weight is to generate pairs or sets of reagents that are substantially chemically identical, but which are distinguishable by mass.

The difference in molecular weight may be brought about by substituting atoms or ligands of the platinum complexes with atoms or ligands of higher or lower molecular weight. Suitable mass-altering ligands include aliphatic groups, carbohydrates, alcohol functionalities, and halogens such as for example F, Cl and Br.

The difference in molecular weight may also be brought about by substituting atoms of the platinum complexes with alternative stable isotopes. Thereto, the platinum complexes may be differentially isotopically labeled, e.g., by substitution of one or more atoms in the complexes with a stable isotope thereof. For example, hydrogens can be substituted with deuteriums, $^{12}C$ with $^{13}C$, $^{14}N$ with $^{15}N$, $^{195}Pt$ with $^{192}Pt$, $^{194}Pt$, $^{196}Pt$ or $^{198}Pt$, $^{16}O$ with $^{18}O$, etcetera. Also P or S atoms present in the complex may be substituted. In preferred embodiments use is made of $^{1}H$ and $^{2}H$, or $^{12}C$ and $^{13}C$, or $^{14}N$ and $^{15}N$, or $^{16}O$ and $^{18}O$, or combinations thereof. Furthermore, using the mixture of platinum isotopes will give a unique pattern of mass distribution making the discerning of labeled entities easier. In yet another alternative embodiment, the marker attached to the platinum complex may provide that complex with a difference in molecular weight by ways as described above. As such, the molecular weight difference may be attributed to (the presence of) the marker.

A(n) (affinity) label may be reacted with the platinum complex at any time. Hence, in accordance with the invention it is possible that a platinum complex is first reacted with a label to obtain a platinum complex-label complex, which is then reacted with the entity, or that the order is reversed. In a preferred embodiment, the platinum complex is first reacted with the label. The term linking complex is used herein to refer to the platinum complex acting as a linking complex between the entity and label, however it is expressly noted that the presence of a label in said platinum complex is not required in all embodiments of the present invention.

Any type of label may be used as long as it can be attached to the platinum complex. Such a label may be a radioactive label, an enzyme , a specific binding pair component such as avidin, streptavidin or biotin, biocytin, iminobiotin, a colloidal dye substance, a phosphorescent label (*e.g.* a europium chelate, a platinum porphyrine), a chemiluminescent label (*e.g.* luminol), a fluorochrome, including a cyanine, a Alexa dye (Molecular Probes), or Bodipy-colourant (Molecular Probes), a rhodamine, dinitrophenol (DNP), carboxyrhodamine, *tert*-butoxycarbonyl, a reducing substance (eosin, erythrosin, *etc.*), a (coloured) latex sol, digoxigenin, a metal (ruthenium), a metal sol or another particulate sol (selenium, carbon and the like), dansyl lysin, a UV dye, a VIS dye, Infra Red Dye, coumarine (*e.g*. amino methyl coumarine), an antibody, protein A, protein G, *etc.*

Particular preferred are DNP, fluorescein, cyanine-colorants and tetramethylrhodamine, *inter alia* because they can form stable complexes with platinum linked to an entity. Other preferred markers include biotin, avidin, streptavidin and digoxygenin.

In one embodiment of the present invention the marker and/or a reactive site of the entity may be connected to platinum through a spacer. Preferably such a spacer comprises a chain having at least four atoms, and preferably not more than 20 atoms, which chain comprises an electron donating moiety on one end and a moiety for reacting with a marker or an entity on the other end, wherein the chain is attached to platinum through the electron donating moiety. Of course, the spacer(s), the marker, the entity and the platinum complex may be attached to each other in any order.

For instance, the spacer(s) may first be attached to the platinum complex followed by reacting the obtained complex with a marker and the entity. It is also possible first to attach the spacer(s) to the marker before the reaction with the platinum complex. The electron donating moiety of the spacer may for example be an amine group or a thiolate anion. Preferably the chain further comprises at least one hetero-atom. Highly preferred spacers are 1,6-diaminohexane and 1,8-diamino-3,6-dioxaoctane. In a preferred embodiment of the invention use is made of 1,6-diaminohexane *tert*-butoxycarbonyl, as an intermediate platinum complex-spacer complex, prior to attaching to a marker and/or entity. In preferred embodiments the spacers differ in molecular weight by substituting atoms as described above, e.g. with alternative isotopes.

The present invention relates to a set of at least two platinum complexes of different molecular weight as described above. Such a set can be used for qualitative and particularly for quantitative analysis of global protein expression profiles in cells and tissues.

In yet another aspect, the present invention relates to a platinum complex comprising at least one different stable isotope as described above.

The invention further relates to an entity labeled with a platinum complex comprising at least one different stable isotope. Preferably, said entity is an amino acid, peptide or protein.

The platinum complexes according to this invention are highly suitable for absolute quantification. This is a method to determine the absolute amounts of entities in a single sample using internal entity standards. The focus in such method is on a specific entity of interest, or its modification state. The method does not require the entity to be labeled, however it does require specific internal standards to be prepared prior to mass spectrometry analysis. Suitable internal standards are entities with similar is not identical features as the entity under investigation.

A further aspect of the present invention relates to a kit of parts comprising a set of said at least two platinum complexes of different molecular weight. Preferably, the difference in molecular weight results from the presence of different stable isotopes in said platinum complexes. The kit of parts may further comprise reaction instructions, one or more test samples, one or more other reagents, one or more test tubes or strips and the like, and one or more preparations selected from the group formed, buffers, marker preparations and preparations for adjusting the ionic strength. Such a kit is very suitable for employing a method according to the invention.

The present invention provides analytical reagents and mass spectrometry-based methods using these reagents for the rapid, and quantitative analysis of proteins or protein function in mixtures of proteins. The analytical method can be used for qualitative and particularly for quantitative analysis of global protein expression profiles in cells and tissues, i.e. the quantitative analysis of proteomes. The method can also be employed to screen for and identify proteins whose expression level in cells, tissue or biological fluids is affected by a stimulus (e.g., administration of a drug or contact with a potentially toxic material), by a change in environment (e.g., nutrient level, temperature, passage of time) or by a change in condition or cell state (e.g., disease state, malignancy, site-directed mutation, gene knockouts) of the cell, tissue or organism from which the sample originated. The proteins identified in such a screen can function as markers for the changed state. For example, comparisons of protein expression profiles of normal and malignant cells can result in the identification of proteins whose presence or absence is characteristic and diagnostic of the malignancy.

The invention will now further be illustrated by the following nonlimiting examples.

EXAMPLES

Example 1

Synthesis of deuterated platinum complexes

Synthesis of platinum complexes is as described by Heeterbrij *et al* (ChemBioChem 2003, 4, 573-583). In one type of Pt complex ethylene-$d_4$-diamine (Sigma-Aldrich Corp. St. Louis, MO, USA) was used to introduce a ) mass difference of 4. From this basic compound the following complexes were synthesized: [platinum(ethylene-$d_4$-diamine) chloride nitrate] and [platinum(ethylene-$d_4$-diamine) (tert-butyl 6-aminohexylcarbamate) chloride], herein referred to as bifunctional ULS-d4 and monofunctional ULS-d4-BOC, respectively. The corresponding light complexes are [platinum (ethylenediamine) chloride nitrate] and [platinum(ethylenediamine) (tert-butyl 6-aminohexylcarbamate) chloride], herein referred to as bifunctional ULS-d0 and monofunctional ULS-d0-BOC, respectively.

Use of deuterated platinum complexes

Reactions of light and heavy platinum complexes with some amino acids and a nucleotide are described, and LC/MS analyses of the resulting reaction mixtures was performed.

Exp #ET320: Reactions of ULS with N-acetyl methionine, 5'-guanosine monophosphate (5'-GMP), *l*-methionine, and N-acetyl cysteine.

Individual solutions as described in table 1 below were prepared in double distilled water and mixed as indicated. The mixtures were allowed to react overnight at room temperature. LC/MS was performed with 100µl of each of the reacted mixtures. For the liquid chromatography a Dionex P580 pump is used, connected to an Alltech Alltima C18 5µm analytical column, with a Gilson 119 UV/VIS detector. The buffers used are (A) 0.1% trifluoro acetic acid (TFA) and (B) acetonitrile. A gradient was used, starting with 95% A and 5% B for 2 minutes, then during 20 minutes a linear gradient to 70% A and 30% B, then in 8 minutes to 10% A and 90% B, followed by returning to starting conditions: 95% A and 5% B. The mass spectroscopic analysis was performed on a ThermoFinnigan AQA spectrometer using Electrospray Interface Ionisation.

Table 1

| Exp #ET320 | a | b | c | d |
|---|---|---|---|---|
| N-acetyl methionine (100mM) | 100µl | 100µl | | |
| 5'-GMP (100mM) | | | 100µl | 100µl |
| Bifunctional ULS-d0 (10mM) | 1000µl | | 1000µl | |
| Bifunctional ULS-d4 (10mM) | | 1000µl | | 1000µl |
| See figure | 1 | 2 | 3 | 4 |

The UV-254 absorption spectra of the LC-chromatogram show that all compounds are eluting within 5 minutes. This means that they have (almost) no retention. It is expected that polypeptides or polynucleotides will have more retention. In each sample the characteristic mass distribution of platinum was observed (194,195,196 and 198 platinum). For the analysis of the products the major mass peak will be referred to herein.

Results

Analysis of the reaction mixtures of N-Acetyl Methionine reveals as the major products:

ET320a1 N-Ac Meth + bifunctional ULS-d0 - HCl = 445.1

ET320a3 N-Ac Meth + bifunctional ULS-d0 = 482.1

ET320b1 N-Ac Meth + bifunctional ULS-d4 - HCl = 449.1

ET320b2 N-Ac Meth + bifunctional ULS-d4 = 486.1

Analysis of the reaction mixtures of 5'GMP reveals as the major product:

ET320c 5'GMP + bifunctional ULS-d0 = 653.1

Furthermore, the product minus HCl is also observed at mass = 617.1

ET320d 5'GMP + bifunctional ULS-d4 = 657.1

And also, this product minus HCl is observed at mass 621.1

Sizes and positions of peaks were essentially as expected. Chemical structures, calculated mass spectra and observed mass spectra are shown in the accompanying figures (1 to 10).

Example 2

Differential mass labeling of a methionine-containing model.tetrapeptide with bifunctional ULS-d4 and bifunctional ULS-d0

This example describes the differential labeling of a tetrapeptide containing one methionine. Also, this peptide was selected with an C-terminal arginine, thus mimicking a tryptic digest.

Materials:

(a) H-Leu-Trp-Met-Arg-OH (LWMR), SERVA #51930, mw 604.8, [1 mg/ml in water] (1.66 mM), stored at -20°C; (b) bifunctional ULS-d0, mw 352.6, [10 mM] in water, stored at 4°C, and (c) bifunctional ULS-d4, mw 356.6, [10 mM] in water, stored at 4°C.

Methods:

16.6 nmol (10 µg) of LWMR was incubated with 50 nmol of either bifunctional ULS-d0 or bifunctional ULS-d4 in a total volume of 100 µl water. Mixtures were allowed to react for 2h at 50°C, unbound platinum complexes were removed from the reaction mixture, and labeled peptides were stored at 4°C until further analysis. For analysis of the reaction products aliquots of the mixtures were diluted 10-fold in water, and subjected to an electrospray ionisation (ESI)-mass spectrometry scan (MS). This was performed in the positive ion mode, using an ESI-triple quad MS (Micromass Ltd., Manchester, UK). Diluted samples were applied by liquid flow injection. For this 10 µl aliquots were manually injected into a 30 µl/min flow of methanol / water (1:1, v/v). Alternatively, nanospray was performed using gold-coated glass microcapillaries. The capillary voltage was set at 1.3 kV and the cone-voltage at 30 V. Data processing was performed using MassLynx software (Micromass Ltd., Manchester, UK). MS/MS was used for identification of the reaction products. In addition, detected reaction products were matched with anticipated reaction products by isotope modeling.

Results:

The used peptide and platinum complexes produced clean and easy interpretable spectrograms with only minor impurities. Free bifunctional ULS-d0 was mainly observed as a 1+ peak at m/z 309.0, in which NO3 was replaced by OH. Under the present reaction conditions (2h at 50°C in water and a 3-fold molar excess of ULS), labeling of LWMR was complete, with no free peptide remaining in the reaction mixtures. The peptide-platinum complex product was present in both 2+ and 3+ forms. Theoretically, the peptide-platinum complex might appear with a departed Cl group (replaced by the peptide; NO3 still present), or with a departed NO3 group (replaced by the peptide; Cl still present). However, the spectra indicate that only the second forms occurs, suggesting that NO3-leaving is preferred above Cl-leaving for the coordinative reaction between Pt and methionine. Nevertheless, two 2+ peptide-ULS-d0 peaks occur, in relatively equal amounts; presumably one with dissociated HCl (m/z 429.9) and the other with HCl still attached to the complex (m/z 448.0). Theoretically bifunctional platinum complexes might induce peptide crosslinking. However, LWMR-platinum complex-LWMR products were not observed under the presently used experimental conditions. Bifunctional ULS-d4 and its reaction products behaved similar to bifunctional ULS-d0, apart from an M/Z shift of 2 up of exactly 2, for 2+ ions.

When aliquots of bifunctional ULS-d0 and bifunctional ULS-d4 labelled LWMR were mixed in a series of predefined ratio's, these LWMR-ULS reaction products appeared as a double set of peaks, of exactly 2 M/Z values apart (for 2+ ions). Most importantly, the relative peak intensities fully reflected the ratio's in which the aliquots had been mixed (figure 11).

Example 3

Differential mass labeling of a methionine-containing model tetrapeptide with monofunctional ULS-d4-BOC and monofunctional ULS-d0-BOC

Materials:

(a) H-Leu-Trp-Met-Arg-OH (LWMR), SERVA #51930, mw 604.8, [1 mg/ml in water] (1.66 mM), stored at -20°C; (b) monofunctional ULS-d0-BOC, mw 569.0 (including NO3), [10 mM] in water, stored at 4°C, and (c) monofunctional ULS-d4-BOC, mw 573.0 (including NO3), [10 mM] in water, stored at 4°C.

Methods:

16.6 nmol (10 μg) of LWMR was incubated with 50 nmol of either monofunctial ULS-d0-BOC or monofunctional ULS-d4-BOC in a total volume of 100 μl water (3 fold molar excess of platinum complex over peptide). Mixtures were allowed to react for 2h at 50°C, unbound platinum complexes were removed from the reaction mixture, and the differentially labeled products were stored at 4°C until further analysis. For analysis of the reaction products aliquots of the mixtures were diluted 10-fold in water, and subjected to an electrospray ionisation (ESI)-mass spectrometry scan (MS). This was performed in the positive ion mode, using an ESI-triple quad MS (Micromass Ltd., Manchester, UK). Diluted samples were applied by liquid flow injection. For this 10 μl aliquots were manually injected into a 30 μl/min flow of methanol / water (1:1, v/v). Alternatively, nanospray was performed using gold-coated glass microcapillaries. The capillary voltage was set at 1.3 kV and the cone-voltage at 30 V. Data processing was performed using MassLynx software (Micromass Ltd., Manchester, UK). MS/MS was used for identification of the reaction products. In addition, detected reaction products were matched with anticipated reaction products by isotope modelling.

Results:

When aliquots of monofunctional ULS-d0-BOC and monofunctional ULS-d4-BOC labeled LWMR were mixed in a series of pre-defined ratio's, these LWMR-platinum complex reaction products appeared as a double set of peaks, at exactly 2 M/Z values apart (for $2^+$ ions). Importantly, the relative peak intensities fully reflected the ratio's in which the aliquots had been mixed (figure 12).

Example 4

The effect of platinum complexes on retention time in LC

Materials:

(a) H-Leu-Trp-Met-Arg-OH (LWMR), SERVA #51930, mw 604.8, [1 mg/ml in water] (1.66 mM), stored at -20°C; (b) monofunctional ULS-d0-BOC, mw 569.0 (including $NO_3$), [10 mM] in water, stored at 4°C, (c) monofunctional ULS-d4-BOC, mw 573.0 (including $NO_3$), [10 mM] in water, stored at 4°C, (d) buffer A (100% MilliQ, 0.15% TFA), and buffer B (95% ACN, 5% MilliQ, 0.15% TFA). Equipment: Shimadzu HPLC SPD-6A; Chart Recorder Kipp & Zonen BD40;
C18, 5u reverse phase column, internal diameter of 4.6mm

Methods:

The column was equilibrated with buffers A and B. Final equilibration was in buffer A. The following gradient was used for analysis of labeled peptide with monofunctional ULS-d0-BOC and monofunctional ULS-d4-BOC:

| Min | %B |
|---|---|
| 0 | 10 |
| 5 | 10 |
| 35 | 50 |
| 40 | 90 |
| 42 | 90 |
| 45 | 10 |
| 45 | STOP |

Absorbance was measured at 220nm and the speed setting of the chart recorder was 5mm/min.
The LWMR was labeled in water at 3 molar excess of platinum complex:peptide and 10 μl samples of the monofunctional ULS-d0-BOC and monofunctional ULS-d4-BOC labeled peptide was applied to the C18 HPLC column.

Results:

The reaction products of the d0 and d4 labeled peptide elute after 28 minutes, with the non labeled peptide eluting after 23 minutes in both cases (Figure 13). The retention times of the products and free peptide are also the same when a mixture of both the d0 and d4 labeled peptides are applied to the column.
To determine whether the labeling buffer had any effect on retention time the experiment was repeated using

LWMR in 25mM Tricine and 25mM $NaNO_3$.

No difference in retention time was observed, the Tricine elutes at the start of the gradient with a retention time of 22 minutes for the peptide. This experiment shows that the retention time on a C18, 4.6mm column are the same regardless of whether the peptide is labeled with the light or heavy platinum complex.

## Claims

1. Use of a platinum complex, having at least one reactive moiety for attachment to a chemical or biological entity, for creating a defined shift in the molecular weight of said entity in order to facilitate mass spectrometric analysis of said entity or of a sample comprising said entity.

2. Use according to claim 1, wherein said platinum complex is represented by one of the following formulas:

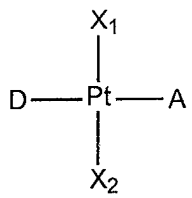

formula 1a

or

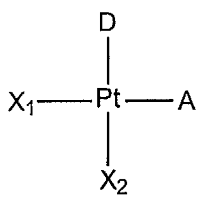

formula 1b

or

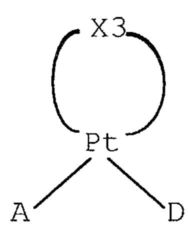

formula 1c

wherein Pt represents the platinum atom, A and D are independently chosen from the group of reactive moieties formed by Cl-, $NO_3$-, $HCO_3$-, $CO_3^{2}$-, $SO_3^{2}$-, $ZSO_3$-, $I^-$, Br-, F- , acetate, carboxylate, phosphate, ethylnitrate, oxalate, citrate, a phosphonate, ZO-, and water, Z being hydrogen or an alkyl or aryl group having from 1 to 10 carbon atoms; wherein X1 and X2 are independently chosen from the group of inert moieties formed by $NH_3$, $NH_2R$, NHRR', NRR'R" groups, wherein R, R' and R" represent an alkyl group having from 1 to 6 carbon atoms;
and wherein X3 represents an inert bidentate moiety as a stabilizing bridge, such as an alkyl diamine, said alkyl preferably having 2 to 6 carbon atoms.

3. Use according to claim 1 or 2, wherein said platinum complex comprises a marker.

4. Use according to claim 3, wherein said a marker and/or a reactive moiety are connected to the platinum moiety of

said platinum complex through a spacer.

**5.** Use according to claim 3 or 4, wherein said marker is a fluorescent label.

**6.** Use according to any one of the preceding claims, wherein said entity is selected from the group consisting of amino acids, peptides, oligopeptide, polypeptides, proteins, immunoglobulins, enzyms, synzyms, phospholipides, glycoproteins, nucleic acids, nucleosides, nucleotides, oligonucleotides, polynucleotides, peptide nucleic acids, peptide nucleic acid oligomers, peptide nucleic acid polymers, amines and aminoglycosides.

**7.** A method for rendering a chemical or biological entity distinguishable by mass spectrometry, said method comprising the step of differentially labeling said entity with at least one platinum complex as defined in any one of claims 1-5.

**8.** A method for mass spectrometric analysis of a chemical or biological entity, said method comprising the steps of differentially labeling said entity with at least one platinum complex as defined in any one of claims 1-5 and analysing said entity by mass spectrometry.

**9.** A method according to claim 7 or 8, wherein at least two entities are labeled and wherein said entities originate from different samples.

**10.** A method according to claim 9, wherein said step of differentially labeling said entity is performed with at least two platinum complexes as defined in any one of claims 1-5, wherein said platinum complexes are of different molecular weight, said difference in molecular weight being ≥1 Da.

**11.** A set of at least two platinum complexes of different molecular weight, wherein each of said platinum complexes is as defined in any one of claims 1-5.

**12.** A kit of parts comprising the set of at least two platinum complexes of different molecular weight as defined in claim 11.

**13.** Kit of parts according to claim 12, further comprising at least one item selected from the group consisting of reaction instructions, test samples, test tubes or strips, buffers, marker preparations and preparations for adjusting the ionic strength.

**14.** A kit of parts for employing a method according to any of the claims 7-10.

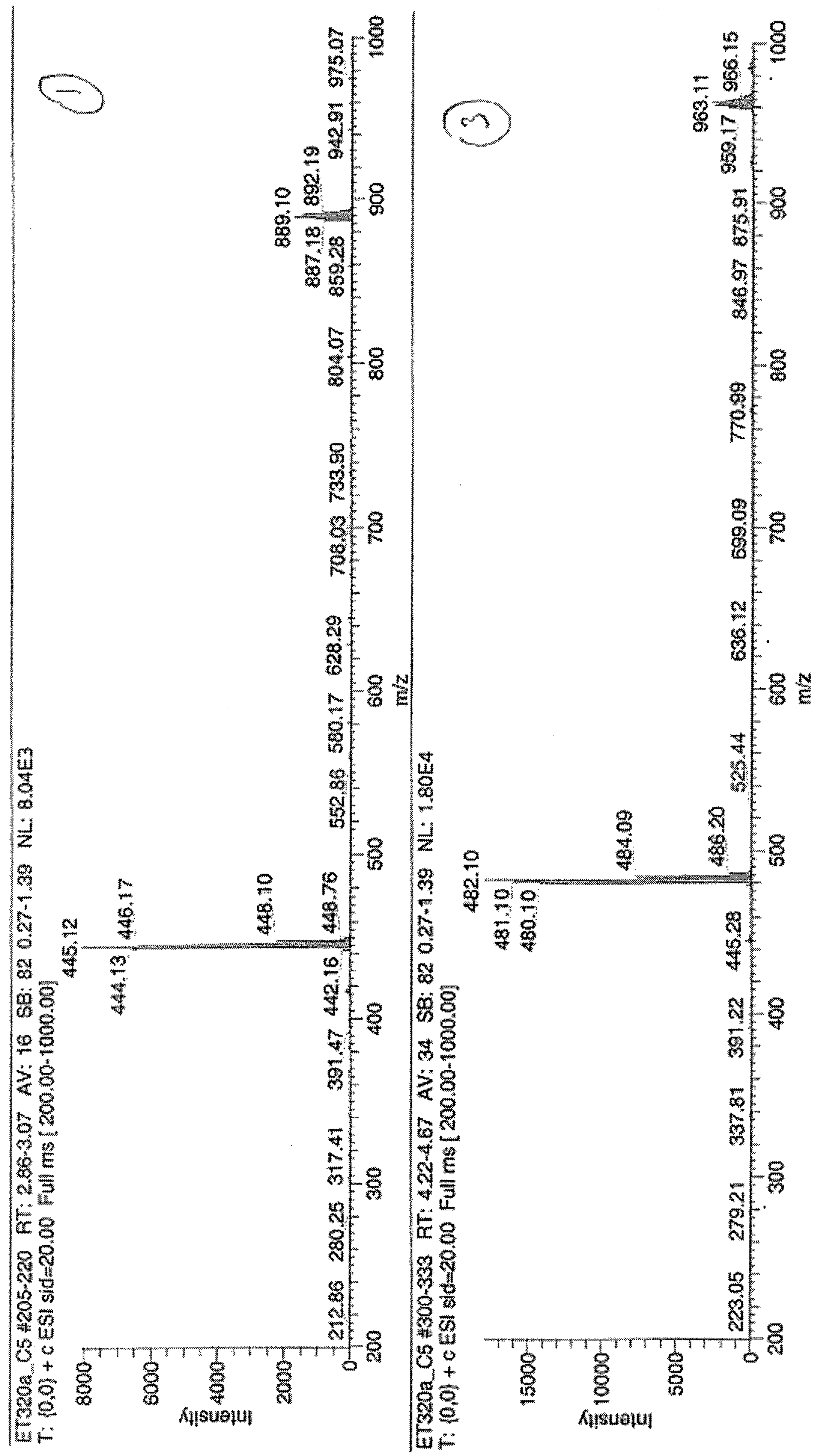


Figure 1: Observed mass spectra ET320a1 & ET320a3

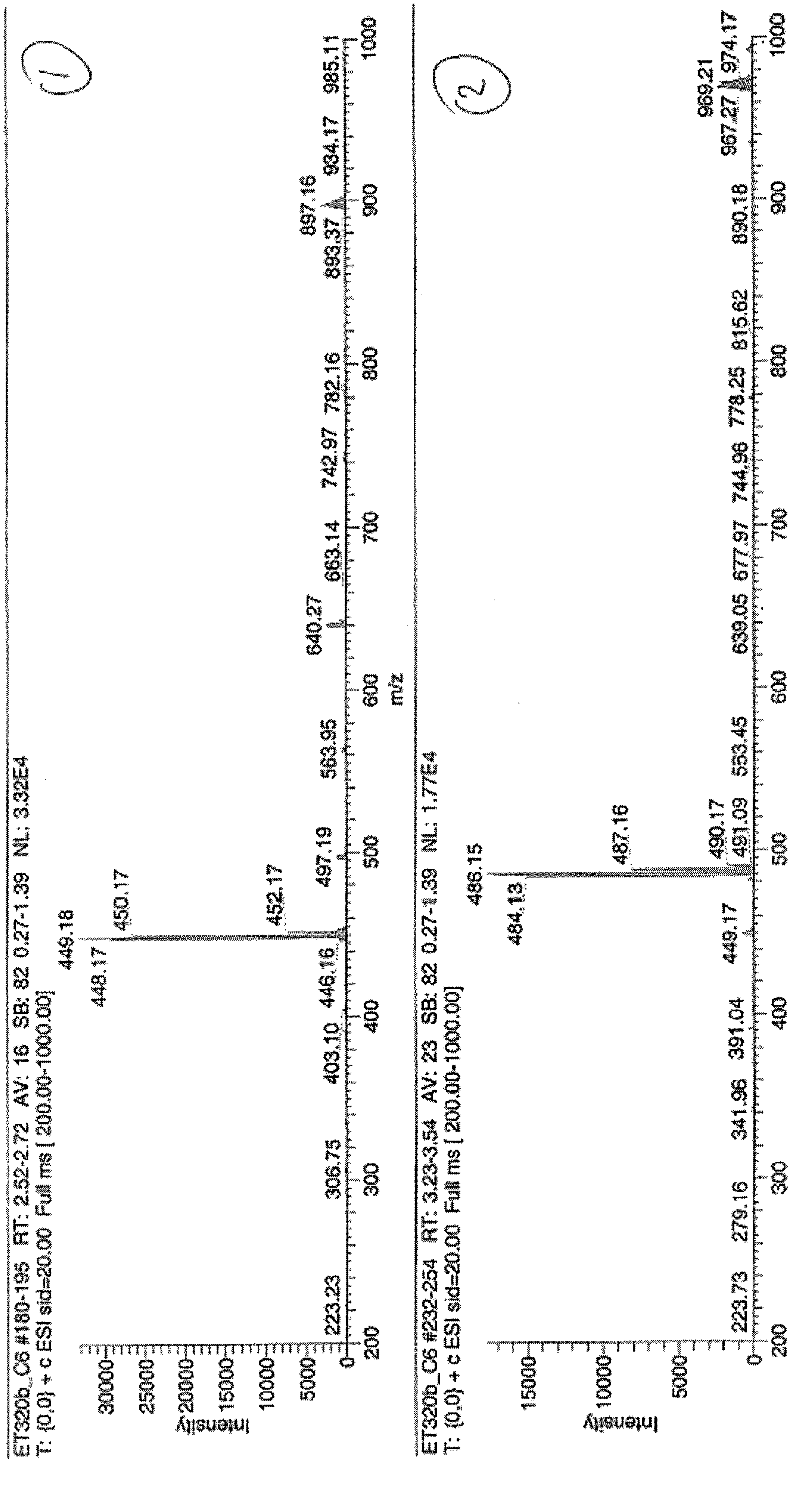


Figure 2: Observed mass spectra ET320b1 & ET320b2

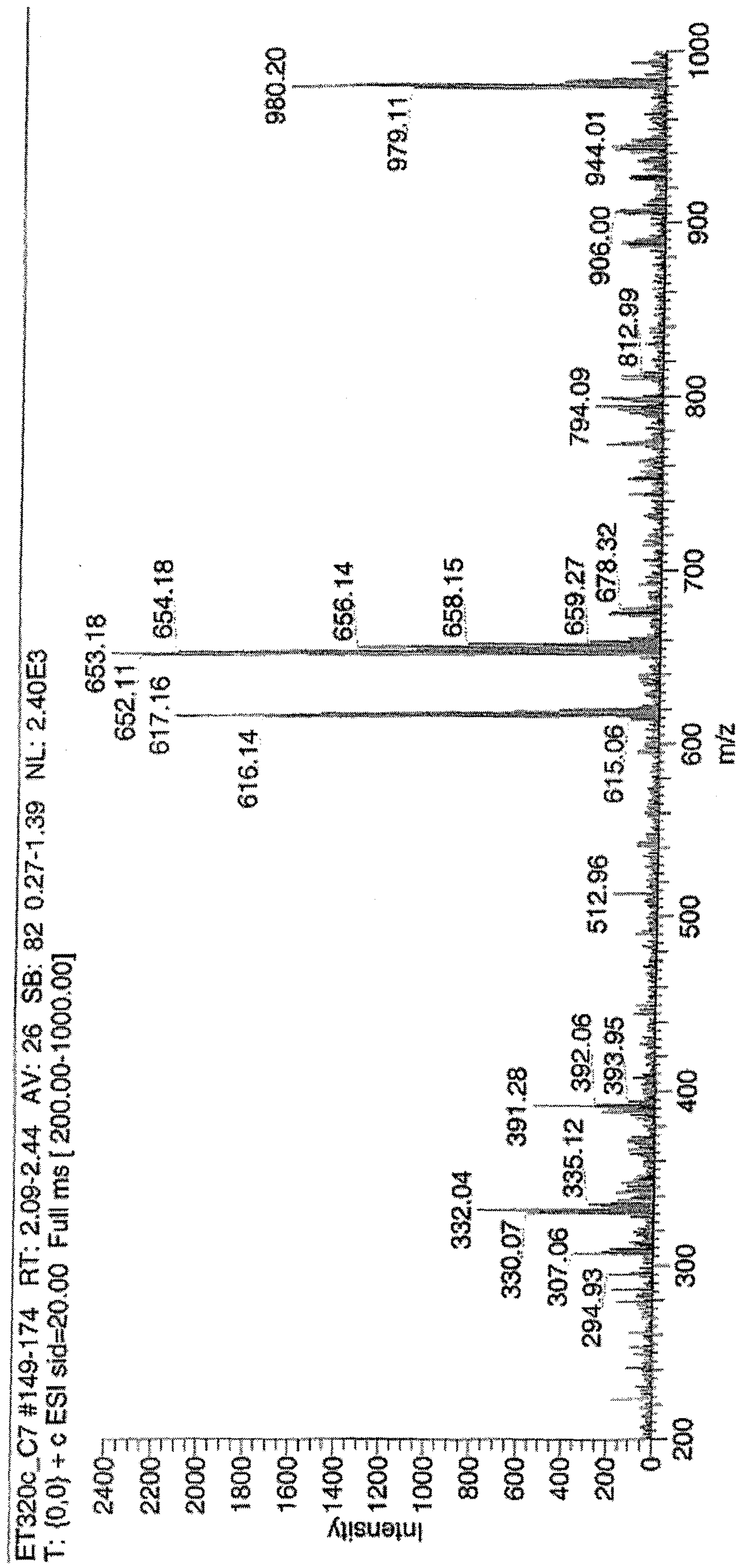


Figure 3: Observed mass spectrum ET320c

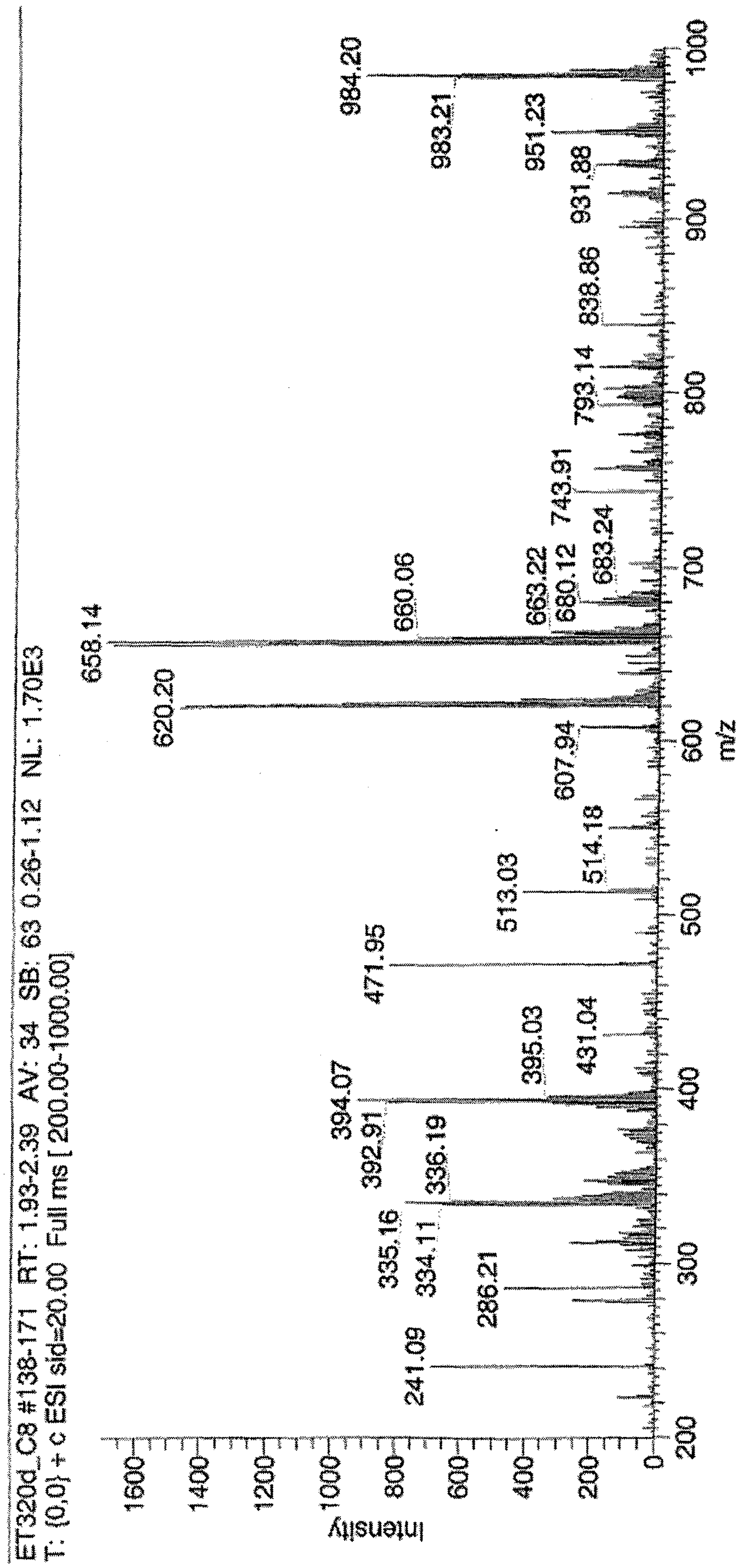


Figure 4: Observed mass spectra ET320d

ET320a1

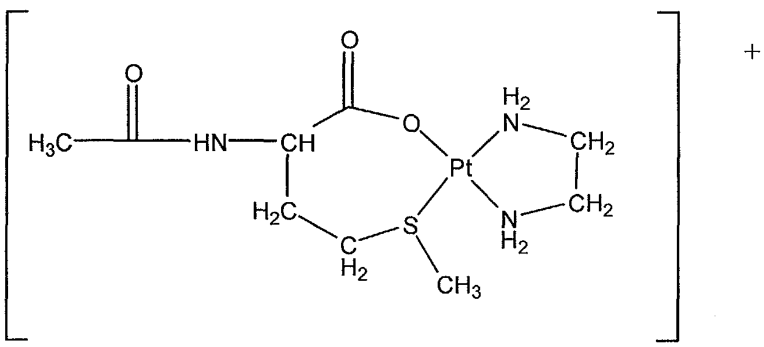

$C_9H_{20}N_3O_3PtS$
Exact Mass: 445.09
Mol. Wt.: 445.42
C, 24.27; H, 4.53; N, 9.43; O, 10.78; Pt, 43.80; S, 7.20

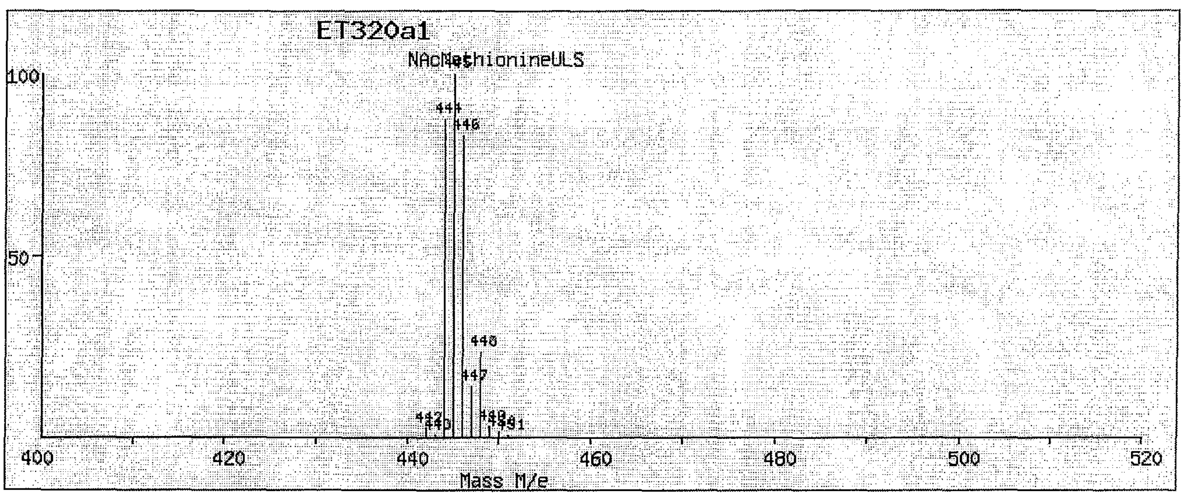


Figure 5: structure and theoretical mass spectrum of ET320a1

ET320a3

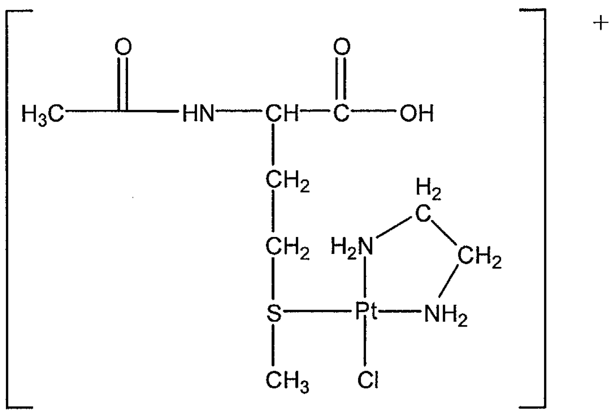

$C_9H_{21}ClN_3O_3PtS$
Exact Mass: 481.06
Mol. Wt.: 481.88
C, 22.43; H, 4.39; Cl, 7.36; N, 8.72; O, 9.96; Pt, 40.48; S, 6.65

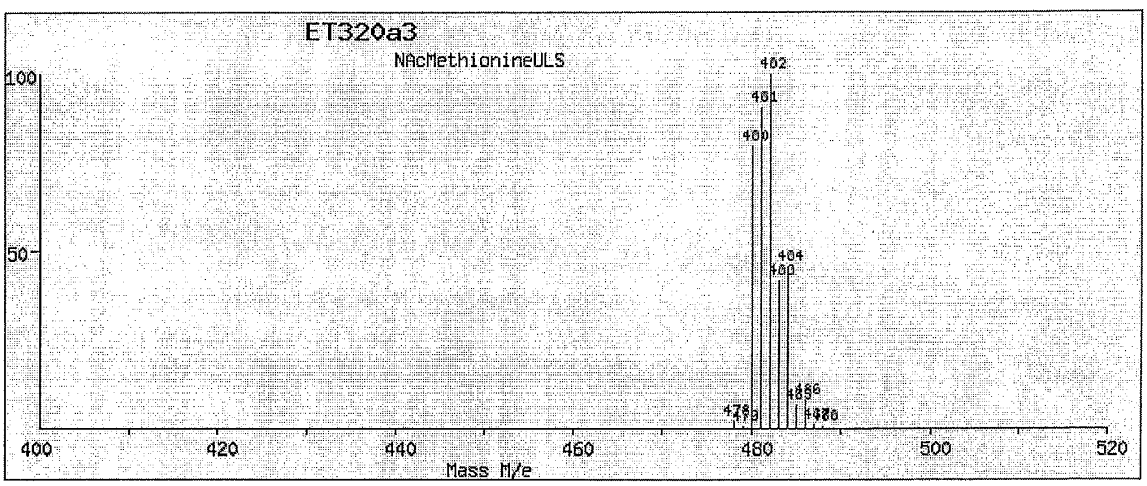


Figure 6: structure and theoretical mass spectrum of ET320a3

ET320b1

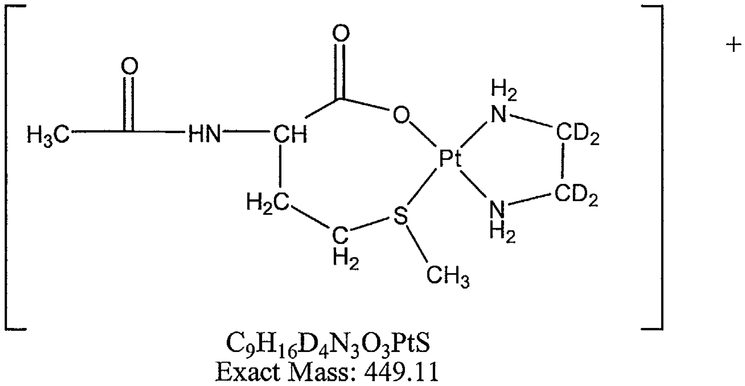

$C_9H_{16}D_4N_3O_3PtS$
Exact Mass: 449.11
Mol. Wt.: 449.44
C, 24.05; H, 5.38; N, 9.35; O, 10.68; Pt, 43.40; S, 7.13

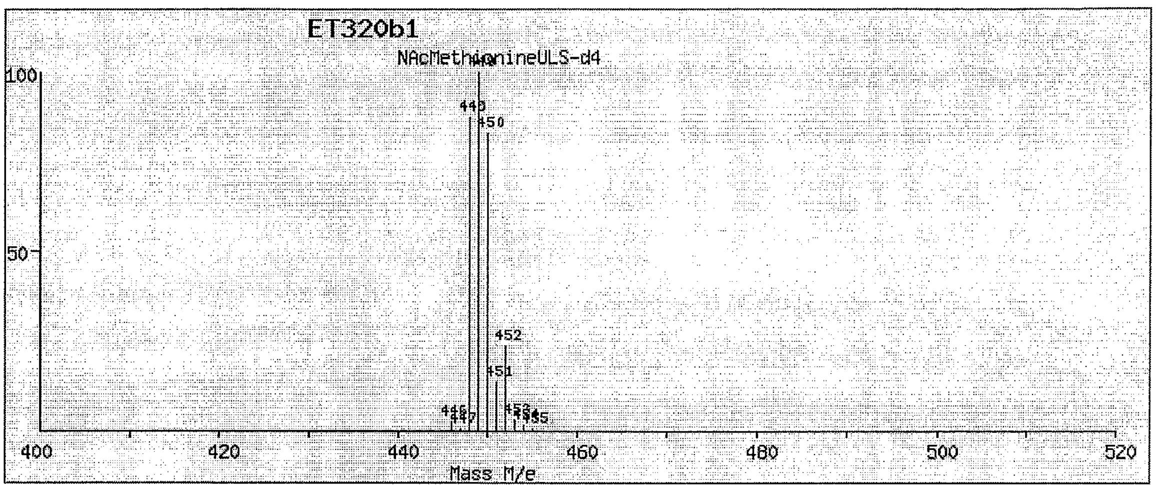


Figure 7: structure and theoretical mass spectrum of ET320b1

ET320b2

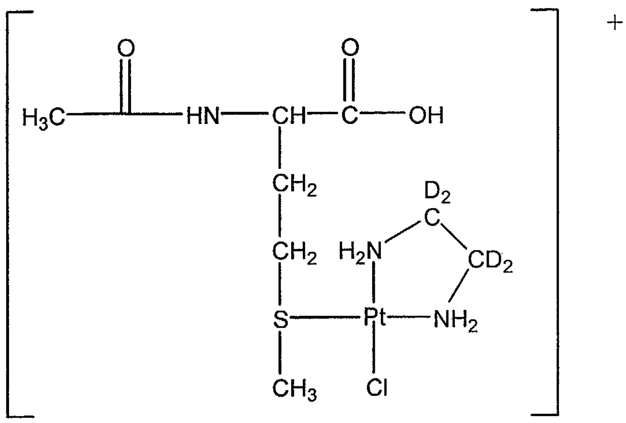

$C_9H_{17}D_4ClN_3O_3PtS$
Exact Mass: 485.09
Mol. Wt.: 485.90
C, 22.25; H, 5.18; Cl, 7.30; N, 8.65; O, 9.88; Pt, 40.15; S, 6.60

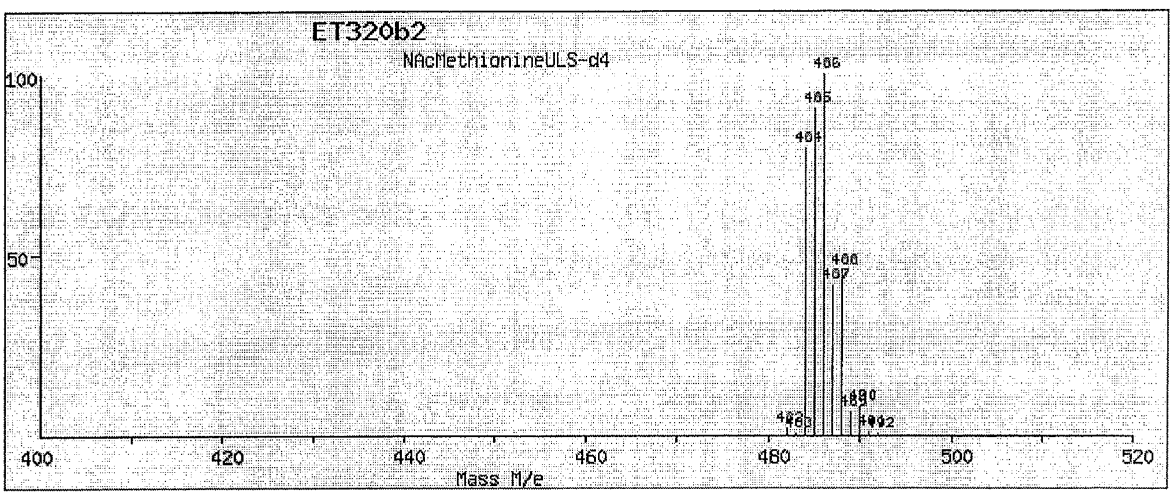


Figure 8: structure and theoretical mass spectrum of ET320b2

ET320c

Molecular Formula = $C_{12}H_{22}ClN_7O_8PPt$

Monoisotopic Mass = 653.060402 Da

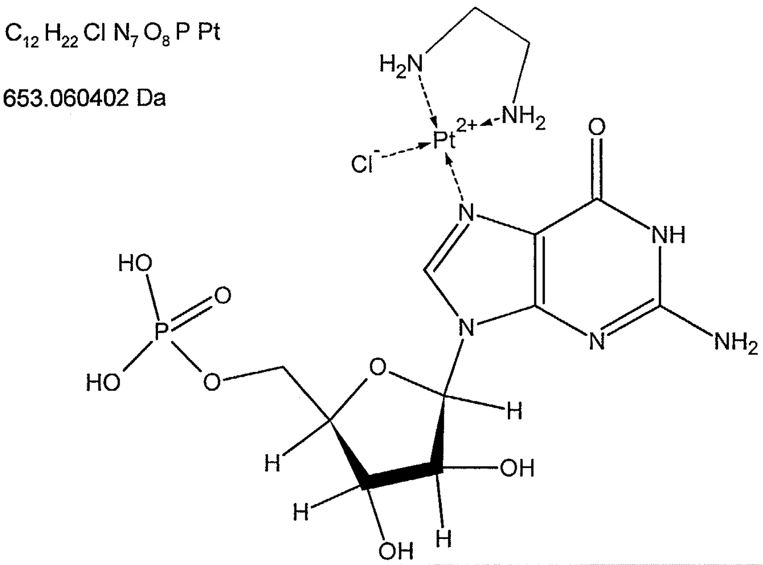

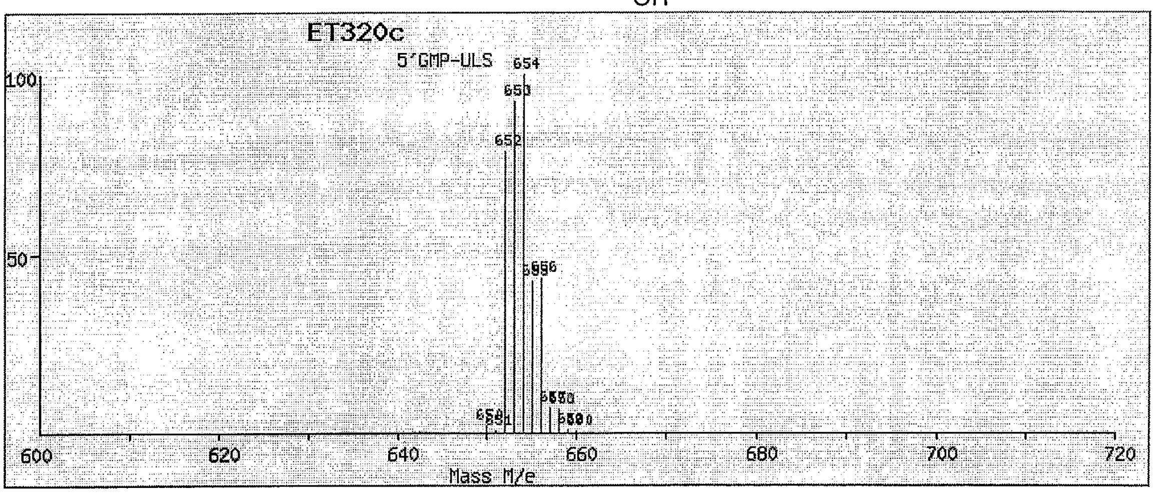


Figure 9: structure and theoretical mass spectrum of ET320c

ET320d

Monoisotopic Mass = 657.08551 Da

Molecular Formula = $C_{12} H_{18} D_4 Cl N_7 O_8 P Pt$

Formula Weight = 657.858

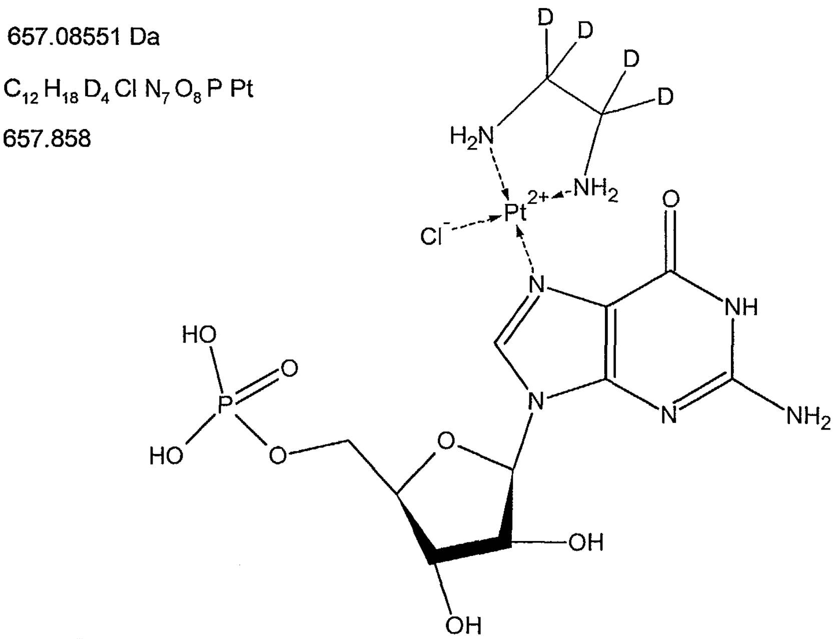

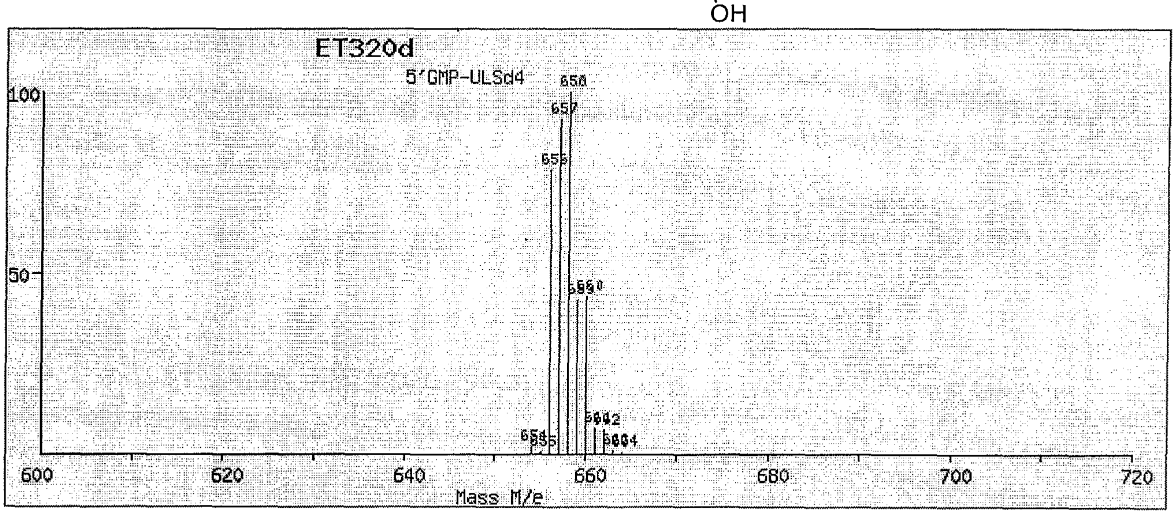


Figure 10: structure and theoretical mass spectrum of ET320d

Figure 11: mass spectrometry analysis of differentially labeled LWMR peptide with light (bifunctional ULS-d0) and heavy (bifunctional ULS-d4) platinum complexes.

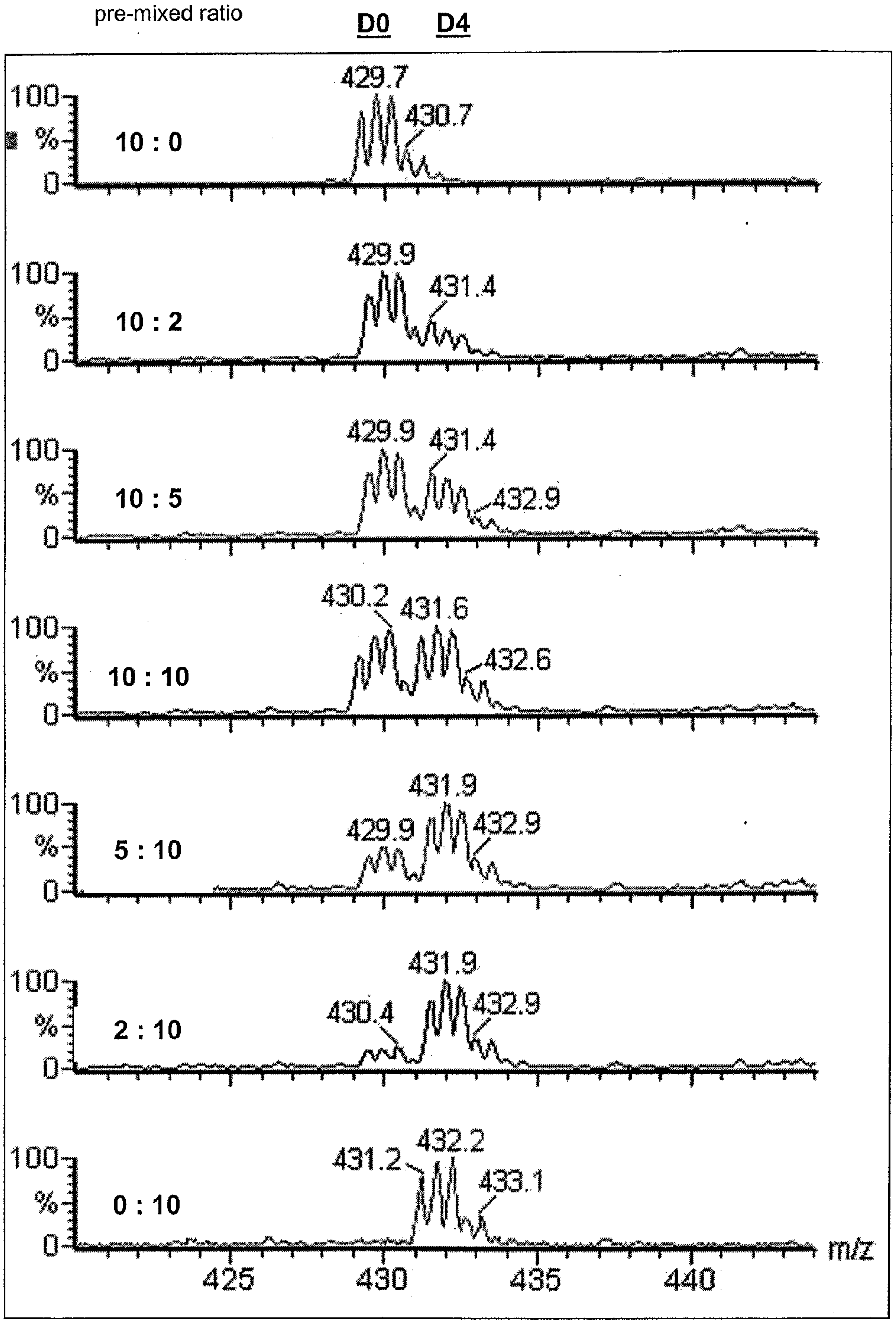


Note that the exact M/Z values of peaks from different spectra do not line up exactly. These minor differences are due to the fact that in some measurements (the 10:0, 10:10 and 0:10 mixtures), nanospray was used for sample application, rather than flow injection.

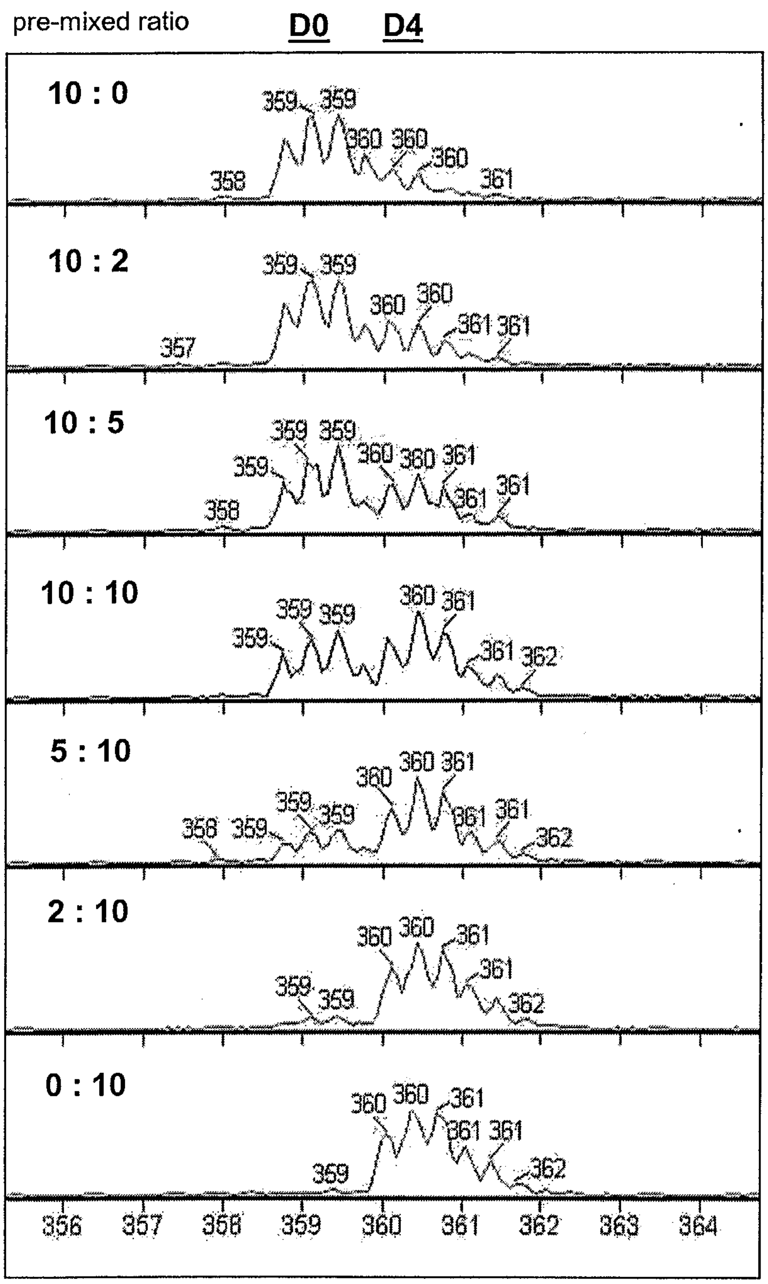


Figure 12: mass spectrometry analysis of differentially labeled LWMR peptide with light (monofunctional ULS-d0-BOC) and heavy (monofunctional ULS-d4-BOC) platinum complexes.

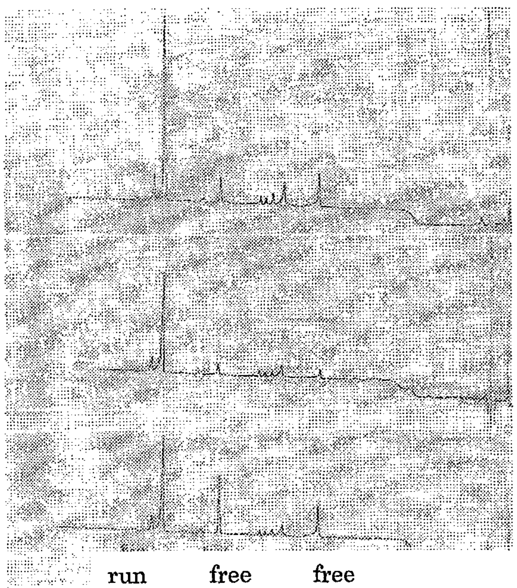


Figure 13: HPLC of LWMR peptide labeled with monofunctional ULS-d0-BOC (37) and monofunctional ULS-d4-BOC (27) and as a mixture. Absorbance was at 220nm on a C18 (4.6mm) 5u column.

European Patent Office

**PARTIAL EUROPEAN SEARCH REPORT**

which under Rule 45 of the European Patent Convention shall be considered, for the purposes of subsequent proceedings, as the European search report

Application Number: EP 03 07 7492

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | JUNICKE HENRIK ET AL: "Synthesis and characterization of novel platinum(IV) complexes with non-functionalized acetylated carbohydrates" JOURNAL OF INORGANIC BIOCHEMISTRY, vol. 81, no. 1-2, 15 July 2000 (2000-07-15), pages 43-48, XP002262376 ISSN: 0162-0134 whole document, in particular fig. 1 --- | 1,3,4,7, 8 | G01N33/84 G01N33/68 |
| X | BERNAREGGI ALBERTO ET AL: "Characterization of cisplatin-glutathione adducts by liquid chromatography-mass spectrometry evidence for their formation in vitro but not in vivo after concomitant administration of cisplatin and glutathione to rats and cancer patients" JOURNAL OF CHROMATOGRAPHY B BIOMEDICAL APPLICATIONS, vol. 669, no. 2, 1995, pages 246-263, XP002262377 ISSN: 0378-4347 whole document, in particular fig's 3-13 --- -/-- | 1-4,6-8 | TECHNICAL FIELDS SEARCHED (Int.Cl.7) G01N C07F |

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 11 March 2004 | Lüdemann, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 03 07 7492

Claim(s) searched incompletely:
1

Reason for the limitation of the search:

Present claim 1 relates to a product defined by reference to a desirable characteristic or property, namely use of a platinum complex "having at least one reactive moiety for attachment to a chemical or biological entity, for creating a defined shift in the molecular weight of said entitiy in order to facilitate mass spectrometric analysis of said entity..."
The claims cover all products having this characteristic or property, whereas the application provides support within the meaning of Article 84 EPC and or disclosure within the meaning of Article 83 EPC for only a very limited number of such products. In the present case, the claims so lack support, and the application so lacks disclosure, that a meaningful search over the whole of the claimed scope is impossible. Independent of the above reasoning, the claims also lack clarity (Article 84 EPC). An attempt is made to define the product by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the use of the products given in claim 2, (see also lack of unity).
The following terms furthermore lack clarity "distinguishable by mass spectrometry" in claim 7, "differentially labeling" in claim 8.

European Patent Office

# PARTIAL EUROPEAN SEARCH REPORT

Application Number
EP 03 07 7492

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | PATENT ABSTRACTS OF JAPAN<br>vol. 2000, no. 04,<br>31 August 2000 (2000-08-31)<br>& JP 2000 002688 A (SUMITOMO CHEM CO LTD;SUMITOMO PHARMACEUT CO LTD),<br>7 January 2000 (2000-01-07)<br>* abstract *<br>--- | 1-4 | |
| X | EP 1 262 778 A (KREATECH BIOTECH BV)<br>4 December 2002 (2002-12-04)<br>* the whole document *<br>--- | 1-14 | |
| X | LUSTIG S ET AL: "DEVELOPMENT OF NATIVE TWO-DIMENSIONAL ELECTROPHORESIS METHODS FOR THE SEPARATION AND DETECTION OF PLATINUM CARRYING SERUM PROTEINS: INITIAL STEPS"<br>FRESENIUS JOURNAL OF ANALYTICAL CHEMISTRY, SPRINGER, BERLIN, DE,<br>vol. 363, no. 5/6, March 1999 (1999-03), pages 484-487, XP009021656<br>ISSN: 0937-0633<br>* the whole document *<br>----- | 1,3-14 | TECHNICAL FIELDS SEARCHED (Int.Cl.7) |

EPO FORM 1503 03.82 (P04C10)

European Patent Office

Application Number
EP 03 07 7492

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

# LACK OF UNITY OF INVENTION SHEET B

Application Number
EP 03 07 7492

The Search Division considers that the present European patent application does not comply with the requirements of unity of invention and relates to several inventions or groups of inventions, namely:

1. Claims: 1-4 (partially)

   Use of a platinum complex according to claim 1 with the formula 1a of claim 2.

2. Claims: 1-14 (partially)

   Use of a platinum complex according to claim 1 with the formula 1b of claim 2.

3. Claims: 1-14(partially)

   Use of a platinum complex according to claim 1 with the formula 1c of claim 2.

## ANNEX TO THE EUROPEAN SEARCH REPORT ON EUROPEAN PATENT APPLICATION NO. EP 03 07 7492

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2004

| Patent document cited in search report | Publication date | Patent family member(s) | Publication date |
|---|---|---|---|
| JP 2000002688 A | 07-01-2000 | NONE | |
| EP 1262778 A | 04-12-2002 | EP 1262778 A1 | 04-12-2002 |
| | | WO 02097439 A2 | 05-12-2002 |
| | | US 2003060647 A1 | 27-03-2003 |

EPO FORM P0459

For more details about this annex : see Official Journal of the European Patent Office, No. 12/82